(19) Europäisches Patentamt
European Patent Office
Office européen des brevets

(11) **EP 4 227 687 B1**

(12) **EUROPEAN PATENT SPECIFICATION**

(45) Date of publication and mention
of the grant of the patent:
**30.07.2025 Bulletin 2025/31**

(21) Application number: **22200490.5**

(22) Date of filing: **10.10.2022**

(51) International Patent Classification (IPC):
**G01N 35/00** (2006.01)      **G01N 35/10** (2006.01)
**G01N 35/04** (2006.01)      **G01N 33/86** (2006.01)
**G01N 33/96** (2006.01)

(52) Cooperative Patent Classification (CPC):
**G01N 35/10; G01N 33/86; G01N 33/96;**
**G01N 35/0092; G01N 35/1002;** G01N 2035/00356;
G01N 2035/0415; G01N 2035/0443;
G01N 2035/0455

(54) **SAMPLE ANALYSIS APPARATUS AND SAMPLE ANALYSIS METHOD**

PROBENANALYSEVORRICHTUNG UND PROBENANALYSEVERFAHREN

APPAREIL D'ANALYSE D'ÉCHANTILLON ET PROCÉDÉ D'ANALYSE D'ÉCHANTILLON

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **28.10.2021 CN 202111266379**
**21.06.2022 CN 202210707458**

(43) Date of publication of application:
**16.08.2023 Bulletin 2023/33**

(73) Proprietors:
• **Beijing Mindray Medical Instrument Co., Ltd.**
**Beijing 102206 (CN)**
• **Beijing Shen Mindray Medical Electronics**
**Technology Research Institute Co., Ltd.**
**Beijing 100085 (CN)**

(72) Inventors:
• **LI, Cong**
**Beijing, 102206 (CN)**
• **LI, Aibo**
**Beijing, 102206 (CN)**
• **GUO, Wenheng**
**Beijing, 102206 (CN)**

(74) Representative: **KIPA AB**
**Drottninggatan 11**
**252 21 Helsingborg (SE)**

(56) References cited:
**US-B2- 10 578 628      US-B2- 11 155 542**

EP 4 227 687 B1

**Description**

TECHNICAL FIELD

The invention relates to sample analysis apparatuses and a sample analysis method.

BACKGROUND

Blood coagulation is a process of blood changing from a liquid state to a gel state. Determination of blood coagulation time is of great significance for mastering blood information and understanding a state of hemostatic function. A mixing plasma correction test is an important means to identify a reason for a prolonged coagulation time when is it confirmed that the blood coagulation time is prolonged. The mixing plasma correction test (also called a mixing study or a correction study) is a screening test for retesting a coagulation time by mixing patient plasma and normal pooled plasma (herein called normal plasma for short) in a certain proportion after excluding the patient from using an anticoagulant. Since an Activated Partial Thromboplastin Time (APTT) test can screen the most types of coagulation factors and coagulation inhibitors, and unexplained APTT prolongation is also the most common in clinical practice, the mixing plasma correction test is mainly applied to APTT, and may also be applied to Prothrombin Time (PT), Thrombin Time (TT), Kaolin coagulation time (KCT) and diluted Russell Viper Venom Time (dRVVT).

Suspected reasons for prolonged coagulation time are mainly divided into two categories: a. congenital coagulation disorder caused by congenital coagulation factor deficiency or abnormality (collectively referred to as coagulation factor deficiency here), moderate and severe patients having a bleeding symptom; and b. acquired coagulation inhibition caused by autoantibodies that inhibit a coagulation response. In the mixing plasma correction test, the congenital coagulation disorder can be corrected by normal pooled plasma, and the coagulation time can be shortened or even restored to a normal level. However, the acquired coagulation inhibition cannot be corrected due to the existence of inhibitors. It is known that the state of patients with the acquired coagulation inhibition will be different due to the difference of autoantibodies. For example, the patient with an autoantibody against the coagulation factor (a coagulation factor antibody, or a coagulation factor inhibitor) may have a bleeding risk or bleeding symptom; and the autoantibody against phospholipid, including a Lupus Anticoagulant (LA), may inhibit the phospholipid necessary for a coagulation process, but the patient often presents a thrombotic symptom. Therefore, it is clinically important to distinguish the coagulation factor inhibitor from the LA. The coagulation factor deficiency, the coagulation factor inhibitor and the LA will all lead to prolonged coagulation time, which is difficult to identify only through a coagulation time screening test.

At present, there are instruments to identify the prolonged coagulation time of the patient through the correction test, but they have the disadvantages such as inconvenience in operation (for example, a sample needs to be incubated manually), poor effect, etc. Patent applications US10578628B2 and US11155542B2 provide teachings related to the technical field of the application.

SUMMARY

In order to solve at least one of the abovementioned problems, the invention provides a sample analysis apparatus as defined by claim 1 and by claim 8 and a sample analysis method according to claim 15. The dependent claims define preferred implementations of the invention.

By designing and introducing a seventh coagulation item into a full-automatic correction test, the sample analysis apparatus and the sample analysis method according to the abovementioned embodiments have a very good effect on identifying, for example, a minute amount of blood coagulation factor inhibitor.

According to the sample analysis apparatus and the sample analysis method provided by the abovementioned embodiments, the initial amount of the plasma sample for the incubation correction test at the beginning of incubation is set to be greater than the amount of the plasma sample for the immediate correction test, and the difficulties of the incubation correction test are overcome by increasing the amount of the incubation sample.

According to the sample analysis apparatus and the sample analysis method provided by the abovementioned embodiments, different detection processes are designed for the immediate correction test and the incubation correction test, a waste of the patient plasma to be detected can be reduced as much as possible, and the utilization rate can be improved.

BRIEF DESCRIPTION OF THE DRAWINGS

FIG. 1 illustrates a schematic structural diagram of a sample analysis apparatus according to an embodiment of the invention.

FIG. 2 illustrates a schematic structural diagram of a sample analysis apparatus according to another embodiment of the invention.

FIG. 3 illustrates a schematic structural diagram of a sample analysis apparatus according to still another embodiment of the invention.

FIG. 4A and FIG. 4B illustrate schematic structural diagrams of reagent carrying components according to two embodiments of the invention, respectively.

FIG. 5 illustrates a schematic structural diagram of a reagent carrying component according to an embodiment of the invention.

FIG. 6 illustrates an example of a first coagulation item to a seventh coagulation item exemplified by taking an APTT correction test as an example.

FIG. 7 illustrates a schematic structural diagram of a sample analysis apparatus according to yet another embodiment of the invention.

FIG. 8 illustrates a schematic structural diagram of a reaction vessel and a buffer vessel according to an embodiment of the invention.

FIG. 9 illustrates an experiment for testing evaporation rates of different incubation sample amounts according to an embodiment of the invention.

FIG. 10 illustrates an experiment for testing evaporation rates of incubation sample amounts under different conditions according to an embodiment of the invention.

FIG. 11 illustrates a schematic structural diagram of a buffer vessel according to an embodiment of the invention.

FIG. 12 illustrates an experiment for testing evaporation rates of different incubation sample amounts according to an embodiment of the invention.

FIG. 13 illustrates a comparison between an automatic test result of a sample analysis apparatus and a result of a manual method according to an embodiment of the invention.

FIG. 14 illustrates an example of a formula editing interface.

FIG. 15 illustrates an example of viewing a default formula and a calculation formula.

FIG. 16 illustrates an example of a report template of a correction test.

FIG. 17 illustrates an example of a result report.

FIG. 18 illustrates a schematic diagram of an immediate correction curve and an incubation correction curve according to an embodiment of the invention.

FIG. 19 illustrates a schematic diagram of an immediate correction curve and an incubation correction curve according to another embodiment of the invention.

FIG. 20 illustrates two schematic diagrams of an immediate correction curve and an incubation correction curve according to an embodiment of the invention.

FIG. 21 illustrates a schematic diagram of areas enclosed by immediate correction curves and incubation correction curves corresponding to heparin, LA positive, hereditary hemophilia, etc. according to an embodiment of the invention.

FIG. 22 illustrates a flowchart of a sample analysis method according to an embodiment of the invention.

DETAILED DESCRIPTION

The invention will be further described in detail below with reference to specific implementations and accompanying drawings. Similar elements in different implementations are represented by associated similar reference signs. In the following implementations, numerous specific details are described to provide a better understanding of the application, and those skilled in the art can easily realize that part features therein can be omitted in certain cases, or can be replaced by other elements, materials, and methods. In certain cases, some operations related to the application are not shown or described in the specification, so as to avoid a core part of the application being overwhelmed by excessive descriptions. It is unnecessary for those skilled in the art to describe these operations in detail, and they can fully understand the relevant operations according to the description in the specification and general technical knowledge in the art.

In addition, characteristics, operations, or features described in the specification may be combined in any appropriate mode to form various implementations. Meanwhile, various operations or actions in the method description can also be changed or adjusted in order in a manner that is apparent to those skilled in the art. Therefore, various sequences in the specification and the accompanying drawings are only for the purpose of clearly describing a certain embodiment, and do not mean that they are necessary sequences, unless otherwise specified that one of the sequences must be followed.

Serial numbers of components herein, such as "first" and "second", are only used to distinguish the described objects without any sequence or technical meaning. Terms like "Connection" and "linkage" mentioned in the application include both direct and indirect connection (linkage), unless otherwise specified.

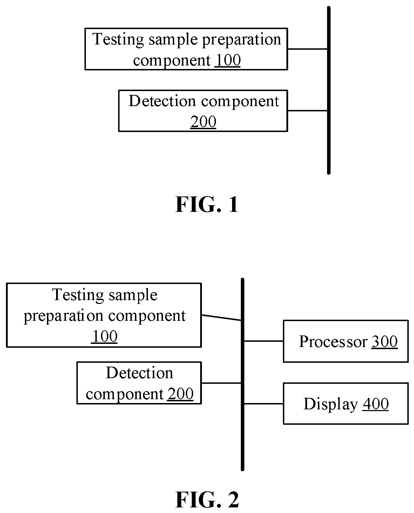

A sample analysis apparatus is disclosed in some embodiments of the invention. Referring to FIG. 1 or FIG. 2, the sample analysis apparatus in some embodiments includes a testing sample preparation component 100 and a detection component 200. In some embodiments, the sample analysis apparatus further includes a processor 300 and may include a display 400. The testing sample preparation component 100 may be configured to prepare a testing sample from a sample or a plasma sample and a reagent. The detection component 200 is configured to detect the testing sample to obtain a detection result, such as a coagulation time of the plasma sample. The processor 300 may be configured to further analyze the detection result to obtain a diagnosis result, for example, a reason for the prolonged coagulation time. The display 400 may be configured to display information. A more detailed description is made below.

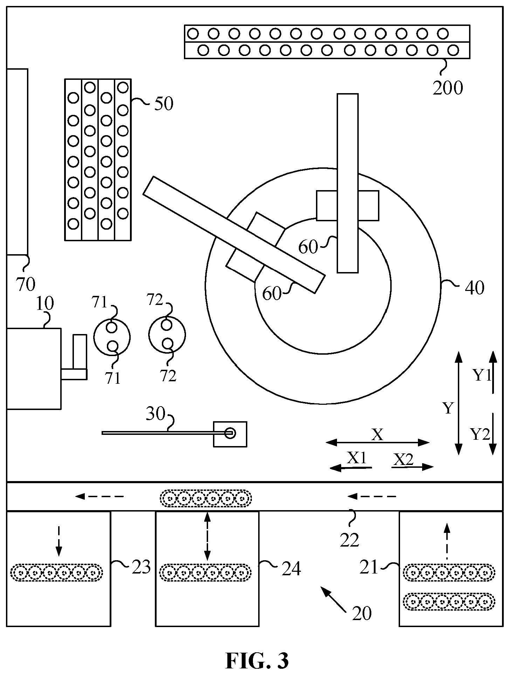

Referring to FIG. 3, in some embodiments, the testing sample preparation component 100 includes a reaction vessel loading component 10, a sample feeding component 20, a sample dispensing component 30, a reagent carrying component 40, a reaction component 50, a reagent dispensing component 60, and a scheduling mechanism 70.

The reaction vessel loading component 10 is configured to supply and load a reaction vessel and/or a buffer vessel. During working, the sample analysis apparatus needs to continuously use empty reaction vessels to complete test items one by one. The sample analysis apparatus prepares a testing sample by adding a sample and a reagent to an empty reaction vessel, and then detects the testing sample through the detection component 200 to obtain the detection result. The reaction vessel loading component 10 may load an empty reaction vessel to a predetermined position, such as a cuvette dispensing position. There may be one or more cuvette dispensing positions. The sample dispensing component 30 is configured to aspirate the sample from the sample feeding component 20 and dispense it into the empty reaction vessel at the cuvette dispensing position.

The sample feeding component 20 is configured to supply a sample rack carrying a sample to be tested. Specifically, the sample vessel carries the sample. The sample rack can hold a plurality of sample vessels. There are a plurality of implementations for the sample feeding component 20. For example, the sample feeding component 20 may include a loading region 21, a sample feeding channel 22, an unloading region 23, and a buffer region 24. A sample aspiration position may be arranged on the sample feeding channel 22. A user may place the sample rack carrying the sample vessel to the loading region 21. The sample rack in the loading region 21 is moved in a direction Y1 in the figure to enter the sample feeding channel 22. The sample rack may move in the sample feeding channel 22 in a direction X1 and pass through the sample aspiration position. When the sample vessel on the sample rack passes through the sample aspiration position (one, two, or more than two sample aspiration positions may be arranged on the sample feeding channel 22), a sample therein can be aspirated by the sample dispensing component 30. After that, the sample rack enters the unloading region 23 through the sample feeding channel 22 in a direction Y2, and the user may take the sample rack out from the unloading region 23. In some other examples, after the sample in the sample vessel on the sample rack is aspirated by the sample dispensing component 30, the sample rack may also enter the buffer region 24 for waiting. When all samples on the sample rack do not need to be retested, the sample rack is scheduled to be transferred to the unloading region 23 for the user to take out. If there is a sample that needs to be retested on the sample rack, the sample rack is scheduled to be transferred back to the sample feeding channel 22 from the buffer region 24. When the sample vessel containing the sample to be retested on the sample rack passes through a retest position (which may be the same position as the abovementioned sample aspiration position) on the sample feeding channel 22, the sample therein may be aspirated by the sample dispensing component 30 for retesting. The sample feeding component 20 is suitable for large-batch sample test occasions. The sample feeding component 20 may be arranged independently from the sample analysis apparatus. When the sample analysis apparatus needs to be connected to a test system in the form of an assembly line, the sample feeding component 20 may be removed directly.

The sample dispensing component 30 is configured to aspirate the sample in the sample vessel at the sample aspiration position and dispense the sample into the reaction vessel and/or the buffer vessel. In some embodiments, the sample dispensing component 30 may include a sampling needle. The sampling needle is driven to move in a two-dimensional or three-dimensional direction by a two-dimensional or three-dimensional driving mechanism. In some embodiments, there may be one or more sampling needles.

The reagent carrying component 40 is configured to carry a reagent. For example, the reagent carrying component 40 may has a plurality of positions for carrying reagent vessels, and the reagent vessels are used to carry reagents. Generally, the reagent carrying component 40 may provide functions, such as refrigerating, for the carried reagent, so as to ensure an activity of the reagent. In some embodiments, the reagent carrying component 40 is arranged in a disc-shaped structure, which has a plurality of positions for carrying the reagent vessels. The reagent carrying component 40 can rotate and drive the carried reagent vessels to rotate, so as to transfer the reagent vessel to a reagent aspiration position, to enable the reagent dispensing component 60 to aspirate the reagent. For example, the reagent carrying component 40 includes a first driving unit configured to drive the reagent carrying component 40 to rotate, and to rotate the reagent vessel to the reagent aspiration position. The reagent carrying component 40 arranged in the disc-shaped structure is specifically described below.

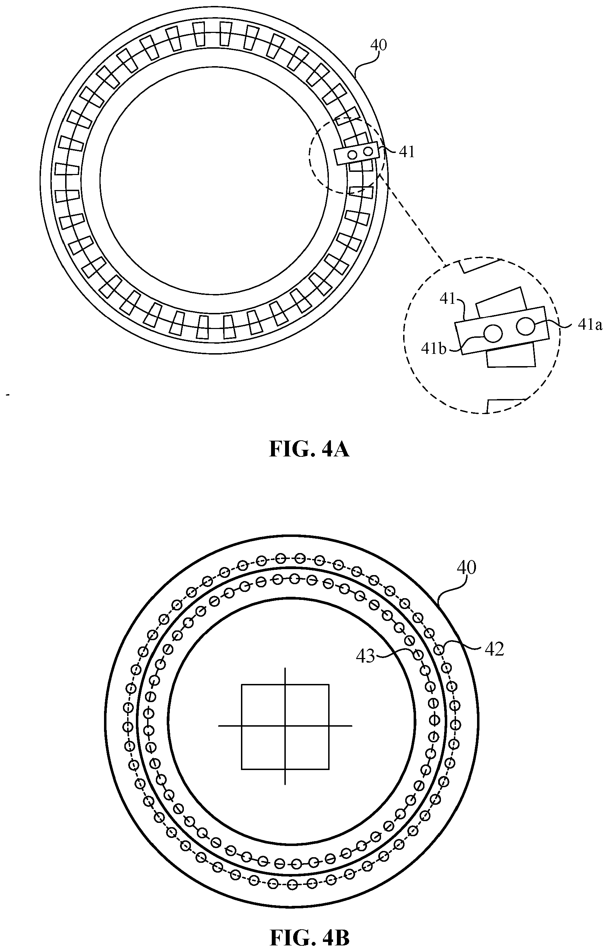

Referring to FIG. 4A, in some specific embodiments, the reagent carrying component 40 is arranged in a disc-shaped structure, which has a plurality of positions for placing linked reagent cups 41. Each reagent cup 41 includes one or more cavity for holding reagents required for item tests. One reagent is placed in one cavity. The reagent carrying component 40 includes a first driving unit configured to drive the reagent carrying component 40 to rotate, and to rotate the cavity of the reagent cup 41 containing the reagent required for an item test to a corresponding reagent aspiration position. In an example, each reagent cup 41 at least includes a first cavity 41a for carrying a first reagent and a second cavity 41b for carrying a second reagent. For example, the reagent cup 41 at least includes a first cavity 41a for carrying a mixed reagent R1 and a second cavity 41b for carrying a trigger reagent R2. The reagent carrying component 40 has a first reagent aspiration position and a second reagent aspiration position different from the first reagent aspiration position. The first driving unit is configured to drive the reagent carrying component 40 to rotate and drive the reagent cup 41 to rotate, so as to rotate the first cavity 41a of the reagent cup 41 to the first reagent aspiration position. The first driving unit is configured to drive the reagent carrying component 40 to rotate and drive the reagent cup 41 to rotate, so as to rotate the second cavity 41b to the second reagent aspiration position.

Referring to FIG. 4B, in some specific embodiments, the reagent carrying component 40 is of a disc-shaped structure, which has a plurality of positions for carrying first reagent vessels 42 containing first reagents, and a plurality of positions for carrying second reagent vessels 43 containing second reagents. The reagent carrying component 40 includes a first driving unit configured to drive the reagent carrying component 40 to rotate and drive the first reagent vessels 42 to rotate, so as to rotate the first reagent vessels 42 to the first reagent aspiration position. The first driving unit is further configured to drive the reagent carrying component 40 to rotate and drive the second reagent vessels 43 to rotate, so as to rotate the second reagent vessels 43 to the second reagent aspiration position. In an example, the reagent carrying component 40 may include a plurality of circles of tracks capable of rotating independently. For example, the reagent carrying component 40 may include two circles of tracks, i.e., an inner circle of track and an outer circle of track. A plurality of positions of the first reagent vessels 42 may be arranged on the outer circle of track, and correspondingly, a plurality of positions of the second reagent vessels 43 may be arranged on the inner circle of track. The inner circle of track and the outer circle of track are driven to rotate independently by the first driving unit.

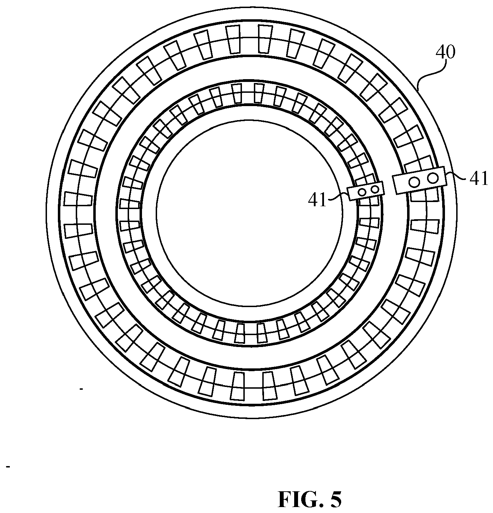

Two types of reagent carrying components 40 are described above. For example, FIG. 4A is an example for placing the linked reagent cups 41, and FIG. 4B is an example of the reagent carrying component 40 including a plurality of circles of tracks capable of rotating independently. Those skilled in the art may understand that the above two examples may be combined. For example, the reagent carrying component 40 may be implemented through a plurality of circles of tracks capable of rotating independently, and at least one circle of track or each circle of track has a plurality of positions for placing the linked reagent cups 41. For example, FIG. 5 illustrates such an example. The reagent carrying component 40 may include two circles of tracks, i.e., an inner circle of track, and an outer circle of track. A plurality of positions for placing the linked reagent cups 41 may be arranged on the outer circle of track. Correspondingly, a plurality of positions for placing the linked reagent cups 41 may also be arranged on the inner circle of track. The inner circle of track and the outer circle of track are driven to rotate independently by the first driving unit.

The above are some descriptions of the reagent carrying component 40. The reagent carrying component 40 may rotate and schedule a corresponding reagent required for a test item to the reagent aspiration position corresponding to the reagent dispensing component 60 in a working period. For example, the first reagent is scheduled to the first reagent aspiration position, and the second reagent is scheduled to the second reagent aspiration position.

The reaction component 50 is configured to incubate a sample, or a reaction liquid formed by mixing a sample and a reagent, or a reaction liquid formed by mixing a sample and a first reagent, or a reaction liquid formed by mixing a sample, a first reagent, and a second reagent. In some embodiments, the reaction component 50 is in a rectangular shape, and has a plurality of vessel placement positions configured to place a reaction vessel and a buffer vessel. Generally, the reaction component 50 may incubate a substance in a vessel at each vessel placement position, for example, incubate the reaction liquid formed by mixing the sample and the reagent in the reaction vessel, and for another example, incubate the sample in the buffer vessel. Specifically, the substance in the vessel may be heated and maintained at $37 \pm 0.5°C$, and specific heating time and heating temperature may be determined by heating parameters corresponding to different detection items.

The reagent dispensing component 60 is configured to aspirate the reagent from the reagent vessel in the reagent carrying component 40 and dispense the reagent into the reaction vessel. The reagent dispensing component 60 may be implemented by a reagent needle. Therefore, in some embodiments, the reagent dispensing component 60 may include a reagent needle. The reagent needle is configured to aspirate the reagent from the reagent carrying component 40 and dispense the reagent into the reaction vessel. In terms of the number of reagent needles, in some embodiments, the reagent dispensing component 60 may be provided with a plurality of reagent needles, and the plurality of reagent needles are arranged in such a way that they can move independently of one another. For example, a group of reagent needles may be provided for the reaction component 50, and a group of reagent needles may be provided for the detection component 200.

The scheduling mechanism 70 is configured to schedule a vessel, such as the reaction vessel and the buffer vessel. For example, the scheduling mechanism schedules to transfer the vessel (the reaction vessel and/or the buffer vessel) among a cuvette dispensing position 71, a standby sample position 72, the reaction component 50, and the detection component 200. There may be one or more cuvette dispensing positions 71, and one or more standby sample positions 72. In addition, there may also be no standby sample position 72.

The detection component 200 is configured to detect a testing sample. In some embodiments, the detection component 200 is an optical detection component, a double-magnetic bead based detection component, or an optical and magnetic integrated detection component. The optical detection component and the optical and magnetic integrated detection component are mainly configured to acquire transmitted light intensity or scattered light intensity of the testing sample, while the double-magnetic bead based detection component is mainly configured to acquire a viscosity of the testing sample.

In some embodiments, the detection component 200 is configured to carry a reaction vessel and detect the testing sample in the reaction vessel. In some embodiments, the detection component 200 is rectangular in shape, and has one or more reaction vessel placement positions.

For some single-reagent test items, the testing sample preparation component 100 may deliver the testing sample to the detection component 200 for detection after a sample and a reagent are added into the reaction vessel. If the testing sample needs to be incubated, the testing sample is scheduled to be transferred to the reaction component 50 for incubating before the testing sample is delivered to the detection component 200 for detection. After incubation is completed, the testing sample is then delivered to the detection component 200 for detection. For some double-reagent test items, the testing sample preparation component may deliver the testing sample to the reaction component 50 for incubating first after the sample and, for example, a first reagent, is added into the reaction vessel. After incubation is completed, the testing sample is then delivered to the detection component 200, then continues to be added with, for example, a second reagent, and then the testing sample is detected.

For a test item or an item that does not need to incubate a sample, the process may be as follows. The sample feeding component 20 schedules a sample vessel containing a sample to be transferred to a sample aspiration position through a sample rack. The sample dispensing component 30 aspirates the sample from the sample vessel, and then dispenses the sample into a reaction vessel at the cuvette dispensing position 71 at this moment. Then, the scheduling mechanism 70 schedules the reaction vessel to be transferred to a reagent position (not shown in the figures, and may be designed inside the reaction component 50 or outside the reaction component 50 as required). The reagent dispensing component 60 aspirates a reagent from the reagent carrying component 40 and dispenses the reagent into the reaction vessel. The mixed liquid in the reaction vessel is scheduled to be transferred to the reaction component 50 by the scheduling mechanism 70, so as to be incubated for a preset duration under a preset condition, and then, the scheduling mechanism 70 schedules the reaction vessel to be transferred to the detection component 200 for detection, or the reagent dispensing component 60 continues dispensing a reagent and then detection is performed.

For a test item or an item that needs to incubate a sample, the process may be as follows. The sample feeding component 20 schedules a sample vessel containing a sample to be transferred to a sample aspiration position through a sample rack. The sample dispensing component 30 aspirates the sample from the sample vessel, and then dispenses the sample into a buffer vessel at the cuvette dispensing position 71 at this moment. In some embodiments, the buffer vessel and the reaction vessel may be the same, and in order to distinguish them functionally herein, one is called a buffer vessel and the other is called a reaction vessel. The buffer vessel generally does not contain a substance, such as a magnetic bead, that is easy to oxidize or may react with plasma. Therefore, in a sample analysis apparatus based on a magnetic bead method, the buffer vessel may be different from the reaction vessel. That is, the magnetic beads in the reaction vessel will be managed to be taken out. Then, the scheduling mechanism 70 schedules the buffer vessel to be transferred into the reaction component 50 for incubating under a preset condition for a preset duration. After incubation is completed, the scheduling mechanism 70 then schedules the buffer vessel to be transferred from the reaction component 50 to the standby sample position 72. The sample dispensing component 30 then aspirates the incubated sample from the buffer vessel at the standby sample position 72 and dispenses the incubated sample into the reaction vessel at the cuvette dispensing position 71 at this moment. Then, the scheduling mechanism 70 schedules the reaction vessel to be transferred to a reagent position. The reagent dispensing component 60 aspirates a reagent from the reagent carrying component 40 and dispenses the reagent into the reaction vessel. The mixed liquid in the reaction vessel is scheduled to be transferred, by the scheduling mechanism 70, into the reaction component 50 for incubating for a preset duration under a preset condition. Then, the scheduling mechanism 70 schedules the reaction vessel to be transferred to the detection component 200 for detection, or the reagent dispensing component 60 continues dispensing a reagent and then the detection is performed. In some embodiments, the buffer vessel, after the sample is aspirated, at the standby sample position 72 may continue to be scheduled back into the reaction component 50 by the scheduling mechanism 70 for use in retesting.

The above is an example of a sample analysis apparatus provided with the standby sample position 72. In some examples, the sample analysis apparatus may also not be provided with the standby sample position 72, but a track is designed in the sample dispensing component 30, so that the sample dispensing component 30 may aspirate a liquid from the reaction component 50 directly. Therefore, for a test item or an item that needs to incubate a sample, one process may also be as follows. The sample feeding component 20 schedules a sample vessel containing a sample to be transferred to a sample aspiration position through a sample rack. The sample dispensing component 30 aspirates the sample from the sample vessel, and then dispenses the sample into a buffer vessel at the cuvette dispensing position 71 at this moment. Then, the scheduling mechanism 70 schedules the buffer vessel to be transferred into the reaction component 50 for incubating for a preset duration under a preset condition. After the incubation is completed, the sample dispensing component 30 aspirates the incubated sample from the buffer vessel in the reaction component 50 and dispenses the incubated sample into the reaction vessel at the cuvette dispensing position 71 at this moment. The scheduling mechanism 70 then schedules the reaction vessel to be transferred to a reagent position. The reagent dispensing component 60 aspirates a reagent from the reagent carrying component 40 and dispenses the reagent into the reaction vessel. The mixed liquid in the reaction vessel is scheduled to be transferred into the reaction component 50 by the scheduling mechanism 70 and is incubated for a preset duration under a preset condition. Then, the scheduling mechanism 70 schedules the reaction vessel to be transferred to the detection component 200 for detection, or the reagent dispensing component 60 continues dispensing a reagent and then the detection is performed. In some embodiments, after a sample is aspirated, the buffer vessel in the reaction component 50 may continue remaining in the reaction component 50 for use in retesting.

The above are some descriptions of the sample analysis apparatus.

The sample analysis apparatus performs an immediate correction test and an incubation correction test.

In some embodiments, the immediate correction test includes the following operations. The testing sample preparation component 100 is configured to prepare a testing sample for the immediate correction test by mixing a plasma sample for the immediate correction test with a reagent for determining a coagulation time. The detection component 200 is configured to obtain a test result, such as the coagulation time, by detecting the testing sample. In some specific embodiments, for the immediate correction test, the testing sample preparation component 100 aspirates a sample from a sample vessel and dispenses the sample into a reaction vessel, so as to prepare the testing sample for the immediate correction test in the reaction vessel.

The immediate correction test and the incubation correction test are further described below.

The plasma sample for the immediate correction test may be one or more of: (1) patient plasma, (2) normal plasma, (3) mixed plasma formed by mixing the patient plasma and the normal plasma in at least one ratio, for example, the ratio of the patient plasma to the normal plasma is 1:4, and/or, 1:1, which will be specifically described below.

In some embodiments, the testing sample preparation component 100 prepares a testing sample for the immediate correction test by mixing patient plasma with a reagent for determining a coagulation time, for example, it may be called a first testing sample. In some specific embodiments, the testing sample preparation component 100 aspirates a sample from a sample vessel containing the patient plasma, dispenses a first amount of sample into a first reaction vessel, and adds the reagent for determining the coagulation time into the first reaction vessel, so as to prepare the first testing sample. The detection component 200 detects the first testing sample to obtain a first coagulation time.

In some embodiments, the testing sample preparation component 100 prepares a testing sample for the immediate correction test by mixing normal plasma and a reagent for determining a coagulation time, for example, it may be called a second testing sample. In some specific embodiments, the testing sample preparation component 100 aspirates a sample from a sample vessel containing the normal plasma, dispenses a second amount of sample into a second reaction vessel, and adds the reagent for determining the coagulation time into the second reaction vessel, so as to prepare the second testing sample. The detection component 200 detects the second testing sample to obtain a second coagulation time.

In some embodiments, the testing sample preparation component 100 prepares a testing sample for the immediate correction test through mixed plasma formed by mixing patient plasma and normal plasma in at least one ratio and a reagent for determining a coagulation time, for example, it may be called a third testing sample. In some specific embodiments, the testing sample preparation component 100 aspirates patient plasma and normal plasma from a sample vessel containing the patient plasma and a sample vessel containing the normal plasma respectively, and dispenses the patient plasma and the normal plasma into a third reaction vessel. The total amount of the samples dispensed into the third reaction vessel is a third amount. The reagent for determining the coagulation time is added into the third reaction vessel to prepare the third testing sample. The detection component 200 detects the third testing sample to obtain a third coagulation time.

In some embodiments, the incubation correction test includes the following operations. The testing sample preparation component 100 prepares a testing sample for the incubation correction test by mixing a plasma sample for the incubation correction test with a reagent for determining a coagulation time. The detection component 200 is configured to detect the testing sample to obtain a test result, such as a coagulation time. The plasma sample for the incubation correction test is a sample or a plasma sample obtained after being incubated for a preset duration under a preset condition. In some specific embodiments, for the incubation correction test, the testing sample preparation component 100 aspirates a sample from a sample vessel, dispenses the sample into a buffer vessel for incubating for a preset duration under a preset condition, then aspirates the incubated sample or plasma sample from the buffer vessel, and dispenses the incubated sample or plasma sample into the reaction vessel, so as to prepare the testing sample for the incubation correction test in the reaction vessel.

The plasma sample for the incubation correction test may be one or more of: (4) patient plasma incubated for a preset duration under a preset condition, (5) normal plasma incubated for a preset duration under a preset condition, (6) mixed plasma formed by mixing the patient plasma and the normal plasma in at least one ratio, for example, the ratio of the patient plasma to the normal plasma is 1:4, and/or, 1: 1, and (7) a sample obtained by mixing the patient plasma incubated for a preset duration under a preset condition and the normal plasma incubated for a preset duration under a preset condition, which may be mixed in at least one ratio, for example, the ratio of the patient plasma to the normal plasma is 1:4, and/or, 1:1, which will be specifically described below.

**In** some embodiments, the testing sample preparation component 100 incubates the patient plasma for a preset duration under a preset condition, and then mixes the incubated patient plasma with a reagent for determining a coagulation time to prepare a testing sample for the incubation correction test, for example, it may be called a fourth testing sample. **In** some specific embodiments, the testing sample preparation component 100 aspirates a sample from a sample vessel containing the patient plasma, dispenses a fourth amount of sample into a fourth buffer vessel, aspirates the sample from the fourth buffer vessel after the sample is incubated for a preset duration under a preset condition, dispenses the incubated sample into a fourth reaction vessel, and adds the reagent for determining the coagulation time into the fourth reaction vessel, so as to prepare the fourth testing sample. The detection component 200 is configured to detect the fourth testing sample to obtain a fourth coagulation time.

In some embodiments, the testing sample preparation component 100 incubates normal plasma for a preset duration under a preset condition, and then mixes the incubated normal plasma with a reagent for determining a coagulation time to prepare a testing sample for the incubation correction test, for example, it may be called a fifth testing sample. In some specific embodiments, the testing sample preparation component 100 aspirates a sample from a sample vessel containing the normal plasma, dispenses a fifth amount of sample into a fifth buffer vessel, aspirates the sample from the fifth buffer vessel after the sample is incubated for a preset duration under a preset condition, dispenses the sample into a fifth reaction vessel, and adds the reagent for determining the coagulation time into the fifth reaction vessel, so as to prepare the fifth testing sample. The detection component 200 is configured to detect the fifth testing sample to obtain a fifth coagulation time.

In some embodiments, the testing sample preparation component 100 incubates mixed plasma formed by mixing patient plasma and normal plasma in at least one ratio for a preset duration under a preset condition, and then mixes the incubated mixed plasma with a reagent for determining a coagulation time to prepare a testing sample for the incubation correction test, for example, it may be called a sixth testing sample. In some specific embodiments, the testing sample preparation component 100 aspirates samples from a sample vessel containing the patient plasma and a sample vessel containing the normal plasma respectively, and dispenses the samples into a sixth buffer vessel. The total amount of the samples dispensed into the sixth buffer vessel is a sixth amount. After being incubated for a preset duration under a preset condition, the sample is aspirated from the sixth buffer vessel and is dispensed into a sixth reaction vessel, and the reagent for determining the coagulation time is added into the sixth reaction vessel to prepare the sixth testing sample. The detection component 200 is configured to detect the sixth testing sample to obtain a sixth coagulation time.

In some embodiments, the testing sample preparation component 100 incubates each of patient plasma and normal plasma for a respective preset duration under a preset condition, mixes the incubated patient plasma and normal plasma in at least one ratio, and then mixes the mixed plasma with a reagent for determining a coagulation time to prepare a testing sample for the incubation correction test, for example, it may be called a seventh testing sample. In some specific embodiments, the testing sample preparation component 100 aspirates a sample from a sample vessel containing the patient plasma, dispenses the sample into one buffer vessel, incubates the sample for a preset duration under a preset condition, aspirates a sample from a sample vessel containing the normal plasma, dispenses the sample into the other buffer vessel, incubates the sample for a preset duration under a preset condition, aspirates the incubated samples from the abovementioned two buffer vessels respectively, dispenses the samples into a seventh reaction vessel, and adds the reagent for determining the coagulation time into the seventh reaction vessel to prepare the seventh testing sample. The detection component 200 is configured to detect the seventh testing sample to obtain a seventh coagulation time.

In the abovementioned test items, the test items for detecting the first testing sample, the second testing sample, the third testing sample, the fourth testing sample, the fifth testing sample, the sixth testing sample, and the seventh testing sample may be called a first coagulation item, a second coagulation item, a third coagulation item, a fourth coagulation item, a fifth coagulation item, a sixth coagulation item, and a seventh coagulation item, respectively.

In some embodiments, the fourth coagulation item and the seventh coagulation item may share or reuse the plasma sample, for example, the two incubate the patient plasma through the same buffer vessel. In some embodiments, the fifth coagulation item and the seventh coagulation item may share or reuse the plasma sample, for example, the two incubate the normal plasma through the same buffer vessel. In one specific embodiment, the fourth coagulation item, the fifth coagulation item, and the seventh coagulation item are performed as follows, or the fourth testing sample, the fifth testing sample, and the seventh testing sample are prepared as follows.

The testing sample preparation component 100 aspirates a sample from a sample vessel containing patient plasma, dispenses the sample into one buffer vessel, such as a fourth buffer vessel, and incubates the sample for a preset duration under a preset condition.

The testing sample preparation component 100 aspirates a sample from a sample vessel containing normal plasma, dispenses the sample into the other buffer vessel, such as a fifth buffer vessel, and incubates the sample for a preset duration under a preset condition.

The testing sample preparation component 100 aspirates the sample from the fourth buffer vessel, dispenses the sample into a fourth reaction vessel, and adds a reagent for determining a coagulation time into the fourth reaction vessel to prepare the fourth testing sample.

The testing sample preparation component 100 aspirates the sample from the fifth buffer vessel, dispenses the sample into a fifth reaction vessel, and adds a reagent for determining a coagulation time into the fifth reaction vessel to prepare the fifth testing sample.

The testing sample preparation component 100 aspirates incubated samples or plasma samples from the fourth buffer vessel and the fifth buffer vessel respectively, dispenses the samples or the plasma samples into a seventh reaction vessel, and adds a reagent for determining a coagulation time into the seventh reaction vessel to prepare the seventh testing sample.

In order to further simplify the process, the testing sample preparation component 100 aspirates an incubated sample from the fourth buffer vessel and dispenses the incubated sample into the fourth reaction vessel and the seventh reaction vessel respectively. The testing sample preparation component 100 aspirates an incubated sample from the fifth buffer vessel and dispenses the incubated sample into the fifth reaction vessel and the seventh reaction vessel respectively.

The above are some descriptions of the immediate correction test and the incubation correction test, or some descriptions of the first coagulation item to the seventh coagulation item. The coagulation items included in the immediate correction test and the incubation correction test may be designed according to a specific purpose, or may be specified by a user through a human-computer interface.

In some embodiments, the immediate correction test at least includes a third coagulation item. The incubation correction test at least includes a sixth coagulation item and a seventh coagulation item. In some other embodiments, the immediate correction test further includes a first coagulation item and/or a second coagulation item. The incubation correction test further includes a fourth coagulation item and/or a fifth coagulation item.

In some embodiments, the immediate correction test includes a first coagulation item, a second coagulation item, and a third coagulation item. The incubation correction test at least includes a fourth coagulation item, a fifth coagulation item, and a sixth coagulation item. In some other embodiments, the incubation correction test further includes a seventh coagulation item.

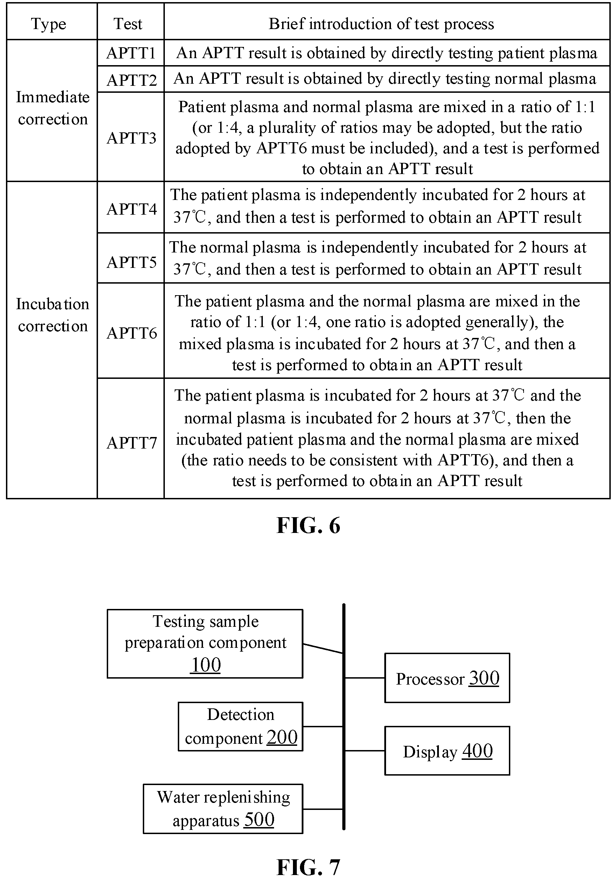

FIG. 6 is an example of a first coagulation item to a seventh coagulation item exemplified by taking an APTT correction test as an example. The first coagulation item to the seventh coagulation item each corresponds to APTT1 to APTT7, respectively.

During an item test, a retest often needs to be performed due to an abnormal result. After an incubated sample is aspirated from a buffer vessel (such as the fourth buffer vessel, the fifth buffer vessel, and the sixth buffer vessel), the testing sample preparation component 100 continues to incubate the sample in the buffer vessel, and the sample in the buffer vessel may be used for a retest. For example, after aspirating the incubated sample from the fourth buffer vessel, the testing sample preparation component 100 continues to incubate the sample in the fourth buffer vessel under a preset condition, so that the sample in the fourth buffer vessel can be used for a retest, for example, the retest of the fourth coagulation item and/or the seventh coagulation item. For another example, after aspirating the incubated sample from the fifth buffer vessel, the testing sample preparation component 100 continues to incubate the sample in the fifth buffer vessel under a preset condition, so that the sample in the fifth buffer vessel can be used for a retest, for example, the retest of the fifth coagulation item and/or the seventh coagulation item. For another example, after aspirating the incubated sample from the sixth buffer vessel, the testing sample preparation component 100 continues to incubate the sample in the sixth buffer vessel under a preset condition, so that the sample in the sixth buffer vessel can be used for a retest, for example, the retest of the sixth coagulation item.

In some embodiments, the amount of the sample or the plasma sample dispensed by the testing sample preparation component 100 into a buffer vessel in an incubation correction test is greater than that of the sample or the plasma sample dispensed by the testing sample preparation component 100 into a reaction vessel in an immediate correction test. In other words, the initial amount of the plasma sample for the incubation correction test at the beginning of incubation is greater than the amount of the plasma sample for the immediate correction test.

For example, the amount of the sample dispensed into the fourth buffer vessel during the preparation of the fourth testing sample is greater than that of the sample dispensed into the first reaction vessel during the preparation of the first testing sample. For another example, the amount of the sample dispensed into the fifth buffer vessel during the preparation of the fifth testing sample is greater than that of the sample dispensed into the second reaction vessel during the preparation of the second testing sample. For example, the total amount of the samples dispensed into the sixth buffer vessel during the preparation of the sixth testing sample is greater than that of the samples dispensed into the third reaction vessel during the preparation of the third testing sample. For example, the total amount of the samples dispensed into two buffer vessels during the preparation of the seventh testing sample is greater than that of the samples dispensed into the third reaction vessel during the preparation of the third testing sample.

In an example in which the fourth coagulation item and the seventh coagulation item do not share or reuse a plasma sample, and the fifth coagulation item and the seventh coagulation item do not share or reuse a plasma sample, the fourth amount is greater than the first amount above, the fifth amount is greater than the second amount above, the sixth amount is greater than the third amount above, and the total amount of the samples dispensed into two buffer vessels during the preparation of the seventh testing sample is greater than the third amount.

In an example in which the fourth coagulation item and the seventh coagulation item share or reuse a plasma sample, and the fifth coagulation item and the seventh coagulation item share or reuse a plasma sample, the sixth amount is greater than the third amount above; the sum of the fourth amount and the fifth amount is greater than the sum of the first amount, the second amount, and the third amount; and the fourth amount is greater than the first amount, and the fifth amount is greater than the second amount.

It is to be noted that no matter the incubation correction test or the immediate correction test, the amount of the sample finally dispensed into the reaction vessel may be a standard amount. This standard amount is a sample amount stipulated or required for performing a test item.

In addition, the amount of the sample herein, such as the first amount, the second amount, the third amount, the fourth amount, the fifth amount, and the sixth amount may be volume and the like, for example, taking uL as a unit.

The agent for determining a coagulation time herein may include one or more reagents, which may be determined according to a test principle and a test method.

The normal plasma herein may be obtained by mixing the plasma samples of not less than 20 normal persons, or by directly purchasing commercial Normal Pooled Plasma (NPP, provided by IL and Stago) or Standard Human Plasma (SHP, provided by Sysmex).

The incubation condition, such as "incubated for a preset duration under a preset condition" described herein may be incubated for half an hour to four hours at a temperature of 30°C to 45°C, for example, incubated for two hours at a temperature of 37°C.

In the description of the first coagulation item to the seventh coagulation item, or the preparation of the first testing sample to the seventh testing sample, the preparation of the testing sample, such as the incubation of a sample, the dispensing of a reagent, the dispensing of the sample, the scheduling of a reaction vessel, the scheduling of a buffer vessel, may refer to the description of a process of a test item or an item that does not need to incubate the sample and the description of a process of a test item or an item that needs to incubate the sample above, which will not be elaborated herein.

Referring to FIG. 7, in some embodiments, the sample analysis apparatus further includes a water replenishing apparatus 500. The water replenishing apparatus 500 is configured to replenish water to a plasma sample during incubation for the incubation correction test according to a preset water replenishing time interval and a preset water replenishing amount, for example, to replenish water to a sample in a buffer vessel. In some embodiments, the water replenishing apparatus 500 is configured to obtain the water replenishing time interval and the water replenishing amount according to an initial amount of the plasma sample for the incubation correction test at the beginning of incubation, for example, the water replenishing time interval and the water replenishing amount are obtained according to the initial amount of the sample in the fourth buffer vessel (the fourth amount above) when water is replenished to the fourth buffer vessel. For another example, the water replenishing time interval and the water replenishing amount are obtained according to the initial amount of the sample in the fifth buffer vessel (the fifth amount above) when water is replenished to the fifth buffer vessel. For another example, the water replenishing time interval and the water replenishing amount are obtained according to the initial amount of the sample in the sixth buffer vessel (the sixth amount above) when water is replenished to the sixth buffer vessel. In some embodiments, the initial amount is positively correlated with the water replenishing time interval, that is, the larger the initial amount is, the larger the water replenishing time interval is. In some embodiments, the initial amount is inversely correlated with the water replenishing amount. In some embodiments, the water replenishing apparatus 500 is further configured to acquire the initial amount above. When it is determined that the initial amount is greater than an initial amount threshold, the water replenishing apparatus 500 does not replenish water.

The water replenishing time interval and the water replenishing amount are mainly correlated with the initial amount during the incubation of the sample, and in addition, the local temperature and humidity and the shape of the buffer vessel may also be taken into account.

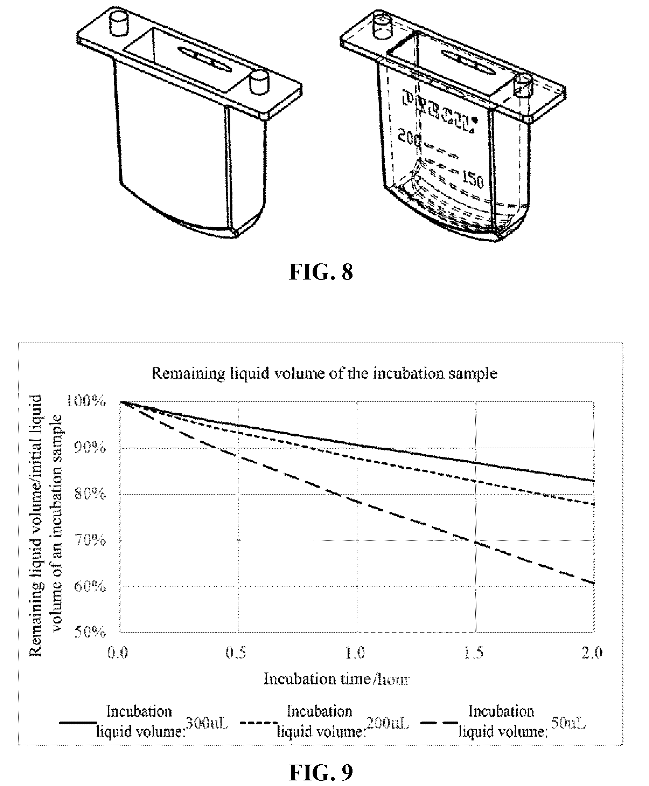

FIG. 8 is an example of a reaction vessel when the detection component 200 is a double-magnetic bead method-based detection component or an optical and magnetic integrated detection component. The left figure therein is a stereoscopic view of the reaction vessel, and the right figure is a corresponding perspective view. A cross section of the reaction vessel in the figure is a rectangle, and a magnetic bead track is arranged at the bottom of the reaction vessel. Magnetic beads are arranged on the track (not shown in the figure). During testing, a driving coil arranged along a long axis direction of the reaction vessel will drive the magnetic beads to swing. Amplitude of the magnetic beads is reduced with an increase of a viscosity of a reaction system, or the magnetic beads even stop swinging. The time when the swinging amplitude of the magnetic beads is reduced to 50% of the initial amplitude is determined as a coagulation time. Since there are magnetic beads in this type of reaction vessel, the magnetic beads will rust in the plasma after a period of time, resulting in a rust interference of the reaction system and prolonged coagulation time. Therefore, the buffer vessel for sample incubation does not contain magnetic beads, that is, the buffer vessel is a reaction vessel with the magnetic beads removed.

A depth-diameter ratio of the abovementioned vessel (the ratio of the depth to the diameter of the vessel, the diameter referring to a diameter of a circumscribed circle of the upper surface) is very small, which is about 1.5. The surface area of liquid is larger, and the surface of the liquid is more easily affected by air flow, so the evaporation rate is higher. Referring to FIG. 9, in an example, after the incubation is performed for two hours on a sample with an amount (such as 50 uL) necessary for a routine test, only 61% of the sample is remained (30.32uL). An APTT or PT reagent is directly added for testing, and the obtained results are both coagulation timeout. When the sample for incubation is increased to 300 uL (the maximum volume of the abovementioned reaction vessel/buffer vessel is 320 uL, and considering the volume occupied by a sample needle when the sample is dispensed, 300 uL is the maximum amount of the sample for incubation), the evaporation rate of the sample is reduced to half of that of 50 uL (the evaporation rate is changed from 39% to 17%). At this moment, the coagulation time can be obtained, but the deviation is large.

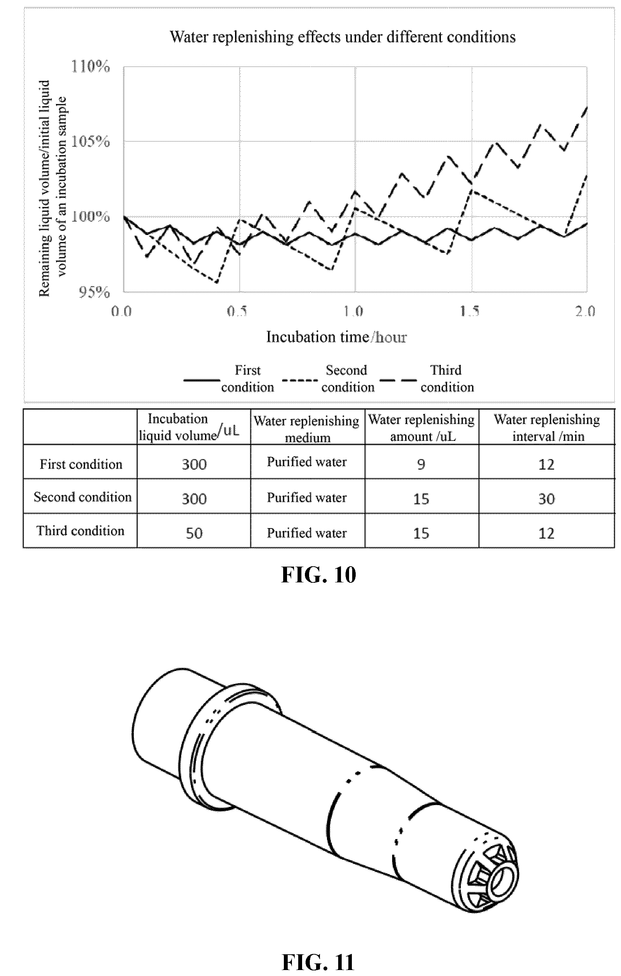

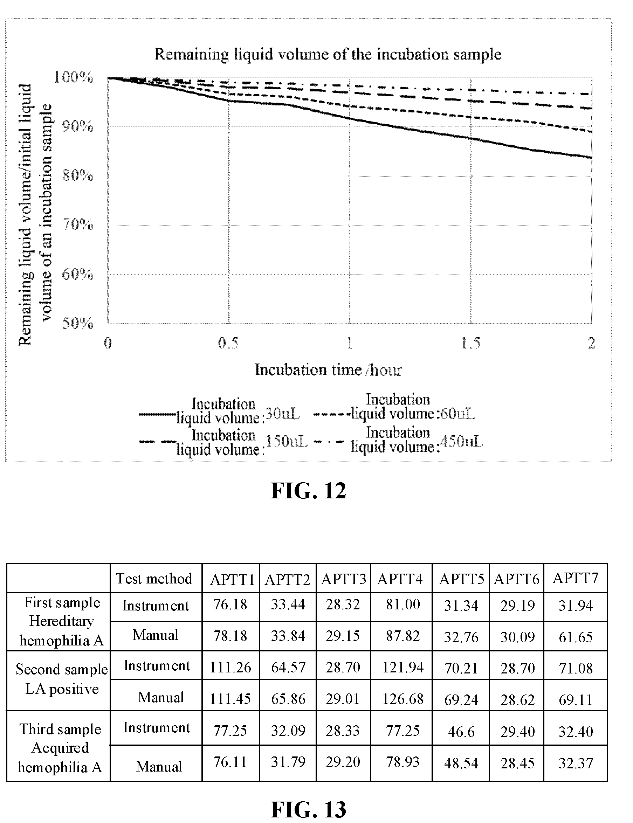

Reference is made to FIG. 10, which illustrates an experiment for confirming whether an evaporation influence can be corrected by using different water replenishment conditions, so as to study the water replenishment effects under different conditions. Under the first condition, the loss caused by an evaporation process can be compensated by a water replenishing process. At this moment, both the repeatability and the accuracy can be consistent with those achieved by a manual method (see FIG. 13). Under the second condition and the third condition, the loss caused by an evaporation process is excessively replenished with water, so that a sample in incubation is diluted, which will lead to the increase of a test result. The main reason for this phenomenon is that: the time interval for replenishing water is too large or the amount of the sample in incubation is too small, and concentration of a salt ion of the sample in incubation exceeds a threshold, resulting in stratification of the sample, the evaporation rate of the incubation sample is inconsistent with that before, and the water replenishment fails. Most of the test results of the samples under such conditions are abnormal or even reported by errors.

Reference is made to FIG. 11, which is another example of a vessel with a large depth-diameter ratio. The evaporation rate of liquid amount in the conventional test (30 uL) has been equivalent to that of the maximum incubation liquid amount of the vessel in FIG. 8 (16.3% and 17.1% respectively, see FIG. 12). After the incubation liquid amount is increased to 150 uL, the evaporation rate within two hours is only 6.2%. At the same time, considering the buffer capacity of APTT reagent itself, the test result will not be affected after the evaporation rate is below 7%. Therefore, the vessel with the large depth-diameter ratio does not require a water replenishing operation, or only needs to be replenished with a small amount of water, and can obtain the same test result as that of the manual method.

Therefore, in some embodiments, the depth-diameter ratio of the buffer vessel is greater than a depth-diameter ratio threshold, for example, the depth-diameter ratio of the fourth buffer vessel is greater than the depth-diameter ratio threshold, the depth-diameter ratio of the fifth buffer vessel is greater than the depth-diameter ratio threshold, and the depth-diameter ratio of the sixth buffer vessel is greater than the depth-diameter ratio threshold. In some embodiments, the depth-diameter ratio threshold is greater than 5, for example, may be 5.6.

In some examples, water does not need to be replenished in some cases where sample incubation is needed. Whether the water needs to be replenished can be determined by identifying the incubation time, for example, the problem about water replenishment does not need to be considered if the incubation time is less than half an hour, and it needs to be considered if the incubation time is greater than half an hour. The specific time threshold may be obtained by a developer according to an evaluation on an evaporation influence of an instrument, which is not necessarily half an hour, and the time thresholds obtained under different conditions such as different instruments, different reagents and different reaction parameters, may be different.

In addition, common reaction vessels and buffer vessels can also be used herein, and the sample evaporation during incubation can be controlled by the operations of increasing the incubation sample amount and replenishing water midway, so as to solve the difficulty of the incubation correction test. Therefore, in some embodiments, compared with manual incubation and solutions of using special vessels for sample incubation and the like, the method for performing the incubation correction test by using the common reaction vessel and buffer vessel have the advantages of a high degree of automation, simple materials, low operational error rate, lower cost and the like.

The above are some descriptions of replenishing water during a sample incubating process for the incubation correction test.

The above are some descriptions involving the immediate correction test and the incubation correction test, or the first coagulation item to the seventh coagulation item.

A full-automatic test process may be as follows.

In a preparation process, a user needs to prepare normal plasma and place it at a designated position, such as the sample rack in the loading region 21, as a sample to be tested before performing the test; or the normal plasma can also be placed on a diluent rack dedicated to a diluent that is used as a "diluent" for patient plasma; or, a fixed position may also be arranged in the sample analysis apparatus for placing the normal plasma. A test order is created in a software interface or through a Laboratory Information System (LIS), and the sample analysis apparatus is in a standby state.

At step 1, the user starts the test, and the sample analysis apparatus will automatically transfer a patient sample to a sample aspiration position. The sample analysis apparatus performs the test according to the test order issued by the user.

At step 2, the sample analysis apparatus determines what type of correction test this test belongs to. For an immediate correction test, the sample analysis apparatus will perform the test according to a general testing process. For an incubation correction test, considering sample evaporation during an incubation process, a transfer loss from a sample vessel to a reaction vessel, testing sample preparation for an automatic retest, etc., the sample amount required for the incubation correction test will exceed the sample amount of the immediate correction test. The step support the preparation of patient plasma only, normal plasma only, mixed plasma of the patient plasma and the normal plasma in a certain ratio. Moreover, "a certain ratio" here is defined before testing, and may be one or more mixing ratios, for example, the ratio of the patient plasma to the normal plasma is 1:4, and for another example, the ratio of the patient plasma to the normal plasma is 1:1.

At step 3, the test is carried out immediately in the immediate correction test, and a reagent is dispensed to obtain a corresponding coagulation time.

At step 4, a sample required in the incubation correction test will be dispensed into a buffer vessel first, and then, will be incubated under a specified condition. The incubation temperature may be 37°C, and the incubation time may be half an hour to four hours, which may be defined by a user. A default value may be two hours. Here, it is to be noted that, incubation needs to be performed by using two buffer vessels in the seventh coagulation item.

At step 5, after the incubation is completed, the buffer vessel will be transferred to a standby sample position, and the sample is aspirated from the buffer vessel at the standby sample position and dispensed into the reaction vessel.

At step 6, the test is carried out in the incubation correction test, and a reagent is dispensed to obtain a corresponding coagulation time.

It can be seen that different test processes are designed for the immediate correction test and the incubation correction test in some embodiments, which not only ensures reliable results of the incubation correction, but also reduces the waste of the dispensing vessels and patient plasma for immediate correction, and improves the test speed. For example, FIG. 13 illustrates a comparison between automatic test results of the sample analysis apparatus herein and test results of a manual method. The instrument in the table refers to the sample analysis apparatus herein. APTT coagulation time results obtained based on three representative patient samples are intercepted. It can be seen that the automatic test results of the sample analysis apparatus are basically consistent with the test results obtained by a manual method. It is to be noted that, the manual method here refers to that a sample is processed manually, and then is tested through related instruments. Specifically, for the immediate correction test, the sample is prepared manually, and the APTT coagulation time thereof is tested by an instrument; and for the incubation correction test, the sample is prepared manually and incubated for two hours in a water-bath box, then the APTT coagulation time of the sample is tested by an instrument.

In some embodiments, different test processes are designed for the immediate correction test and the incubation correction test, which can reduce the waste of the patient plasma to be tested as much as possible and improve the utilization rate. In addition, the amount of a liquid in the buffer vessel is determined by the evaporation requirements, which not only further reduces the waste of the patient plasma, but also can prepare a sample for an automatic retest, thereby improving the automatic retest speed of the incubation correction test, and shortening a reporting time.

In some embodiments, after the detection component 200 obtains a test result, such as the coagulation time, the processor 300 is configured to obtain a reason for the prolonged coagulation time of the patient plasma at least according to the sixth coagulation time and the seventh coagulation time. For example, the processor determines, according to the sixth coagulation time and the seventh coagulation time, whether the prolonged coagulation time is caused by the existence of a time-independent inhibitor or a time-dependent inhibitor in the patient plasma, and generates a corresponding prompt. In some embodiments, the display 400 may be configured to display the prompt.

In some other embodiments, the processor 300 is configured to obtain a reason for the prolonged coagulation time according to the third coagulation time, the sixth coagulation time, and the seventh coagulation time. For example, when the third coagulation time is indicated as corrected, the sixth coagulation time is indicated as corrected, and the seventh coagulation time is indicated as corrected, the processor 300 determines that the reason for the prolonged coagulation time of the patient plasma is the deficiency of a coagulation factor and the absence of an inhibitor, and generates a corresponding prompt. When the third coagulation time is indicated as corrected, the seventh coagulation time is indicated as corrected, and the sixth coagulation time is indicated as uncorrected and the prolonged time exceeds a critical value, the processor 300 determines that the reason for the prolonged coagulation time of the patient plasma is the existence of a time and temperature dependent inhibitor, and generates a corresponding prompt. When the third coagulation time is indicated as uncorrected, the sixth coagulation time is indicated as uncorrected, the seventh coagulation time is indicated as uncorrected, and the prolonged time of the sixth coagulation time is longer than that of the seventh coagulation time by no more than a critical value, the processor 300 determines that the reason for the prolonged coagulation time of the patient plasma is the existence of a time and temperature-independent inhibitor, and generates a corresponding prompt. When the third coagulation time is indicated as uncorrected, the sixth coagulation time is indicated as uncorrected, and the seventh coagulation time is indicated as uncorrected, and the prolonged time of the sixth coagulation time is longer than that of the seventh coagulation time by more than the critical value, the processor 300 determines that the reason for the prolonged coagulation time of the patient plasma is the existence of a time and temperature dependent inhibitor, and generates a corresponding prompt.

Some default formulas of the sample analysis apparatus may be used when the reason for the prolonged coagulation time is determined according to the coagulation time obtained from the immediate correction test and the incubation correction test. Taking the correction test of APTT as an example, there may be the following default formulas.

$$\text{Formula 1: } RI = (APTT\,3 - APTT2) / APTT\,1 \times 100\%.$$

$$\text{Formula 2: } APTT\,6 - APTT\,7.$$

In addition, after the correction test confirms the existence of the coagulation factor inhibitor, Nijmegen method and Bethesda method can be used to determine a potency of the inhibitor (unit: BU/mL). In Chinese guidelines for diagnosis and treatment of coagulation factor VIII/IX inhibitors (2018 Edition), the Bethesda method is determined as a standard method for testing the titer of a FVIII/FIX coagulation factor inhibitor. A specific operation is that: patient plasma is incubated for two hours at 37°C after being successively diluted in multiple proportions and mixed with the normal plasma, a residual activity of coagulation factors in various samples is tested and is compared with activity of a standard coagulation factor to obtain a ratio, a dilution ratio corresponding to the ratio close to 50% is found, and its reciprocal is the potency (that is, the titer) of the coagulation factor inhibitor. The sample needs to be incubated for two hours during the process, and the activity of coagulation factors at a plurality of samples needs to be tested, so time and labor are wasted, the repeatability is low, and the degree of standardization is low.

A large number of hospitals have carried out a lot of work and accumulated a large amount of data. For example, a hospital introduced a novel test method when studying the activity of FVIII coagulation factor inhibitor, which has the following advantages compared with a traditional method.

The test of the activity of the FVIII coagulation factor inhibitor and the APTT correction test are tested synchronously, which realizes that screening and diagnosis are carried out synchronously.

The potency of the FVIII coagulation factor inhibitor is determined within 20 min, and a large amount of time is saved compared with a 2-hour incubation process of the Bethesda method.

The whole process is automated. The 2-hour automatic incubation process of the Bethesda method has not been supported by a manufacturer. However, the above method provided by the hospital can be fully automated, which not only saves manpower, but also improves the repeatability and the accuracy.

However, there is no fully automatic coagulation analyzer that can support a user to carry out the abovementioned study process, and medical workers can only summarize and sort out data through daily office software.

Therefore, in some embodiments, the user can also perform further calculation and analysis on the test results through a self-defined formula. Therefore, in some embodiments, the display 400 displays a formula editing interface, which is used to display the calculation formula input by the user through an input component (not shown in the figure). The input component may be a mouse, a keyboard, etc., or the input component may also be integrated into the display 400, so that the display 400 may be a display screen with a touch function. The processor 300 obtains the abovementioned calculation formula and the test results involved in the abovementioned calculation formula (such as one or more of the first coagulation time to the seventh coagulation time), to obtain calculation results of the abovementioned calculation formula. The display 400 also displays a calculation result. In some embodiments, the display 400 also displays a diagnostic result associated with the calculation result, which can be the reason of the prolonged coagulation time, such as "the existence of anticoagulant drug interference is suspected", "the titer of the coagulation factor inhibitor > 3.5 BU/mL", etc. The diagnosis result may be obtained by the processor 300 according to the abovementioned calculation result, or input by the user through the input component.

An example of two user-defined calculation formulas is as follows.

$$\text{Formula 3: } APTTD = APTT6 - APTT3;$$

$$\text{Formula 4: } APTTCI = APTT1 \times APTT3 \times APTT6 \times APTTD^3/10^6.$$

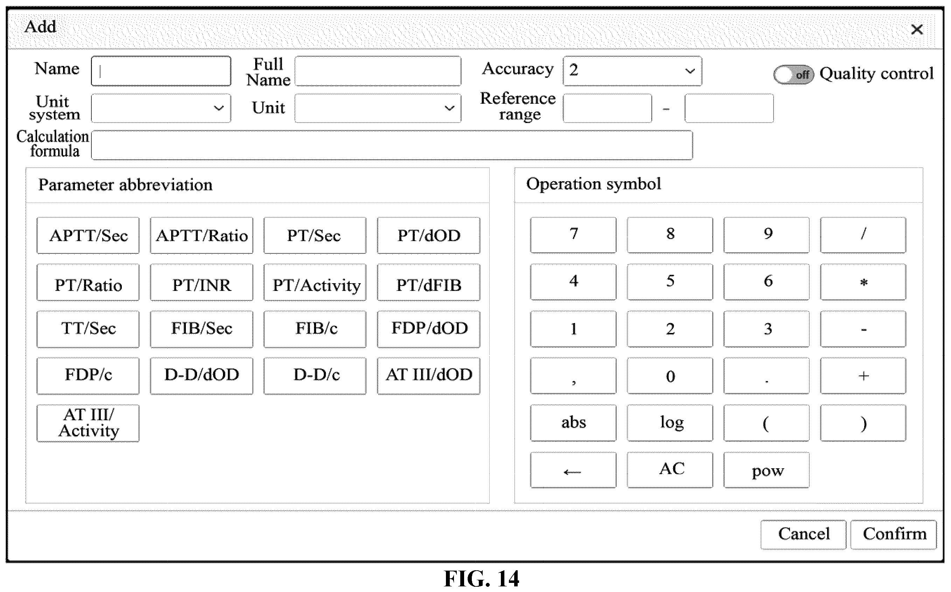

FIG. 14 illustrates an example of a formula editing interface. In some embodiments, the formula editing interface includes: an editing item for a name of a calculation formula or a name of a calculation result, and a formula editing item for a calculation formula.

The editing item for the name of the calculation formula or the name of the calculation result is configured for a user to input the name of the calculation formula or the name of the calculation result through the input component.

The formula editing item for the calculation formula is configured to input the calculation formula through the input component.

In some embodiments, the formula editing interface further includes at least one of: a unit editing item for a calculation formula, an accuracy editing item for the calculation result of the calculation formula, or a reference range editing item for the calculation result of the calculation formula.

The unit editing item for the calculation formula is configured for a user to input the unit of the calculation formula through the input component.

The accuracy editing item for the calculation result of the calculation formula is configured for a user to input the accuracy of the calculation result through the input component.

The reference range editing item for the calculation result of the calculation formula is configured for a user to input the reference range of the calculation result through the input component.

The formula editing interface may further be configured to provide one or more detection result items capable of participating in calculation, so that the user can input a detection result represented by the selected detection result item into the formula editing item through the input component, for example, APTT/Sec included in the "parameter abbreviation" item in the figure.

The formula editing interface may further be configured to provide one or more operation symbol items for editing a formula, so that the user inputs an operation symbol represented by the selected operation symbol item into the formula editing item through the input component, for example, numbers and symbols included in the "operation symbols" item in the figure.

It is to be noted that, in some examples, the formula editing interface allows to use the test results that are not limited to the APTT correction test. It can be understood that, in some embodiments, the calculation formula can only be carried out when all the test results involved in the calculation are valid. The "valid" here includes two meanings: first, when the test item is performed, a clinical research system cannot actively initiate an associated test, and all test items need to be initiated by a user; and second, the test has been completed and normal results have been obtained. The result of the test that was not completed due to instrument failure or the dilution is needed because a result is beyond of a linear range are not considered as normal results.

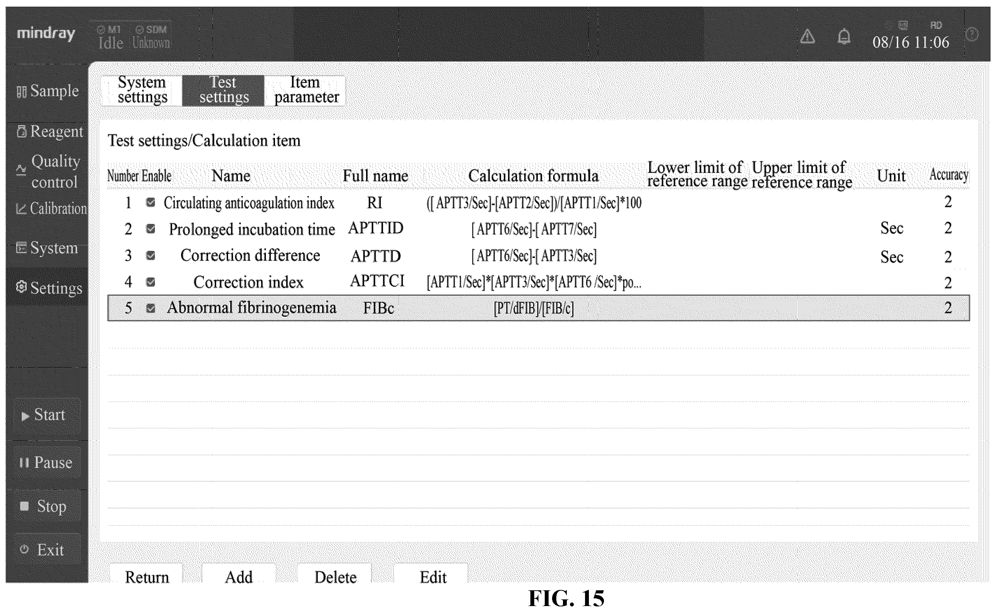

In some embodiments, the display 400 further provides an interface for a user to view the default formula and the calculation formula input by the user. FIG. 15 is an example.

In some embodiments, the processor 300 is further configured to obtain a correction test report template and generate a result report according to the correction test report template and the abovementioned calculation results.

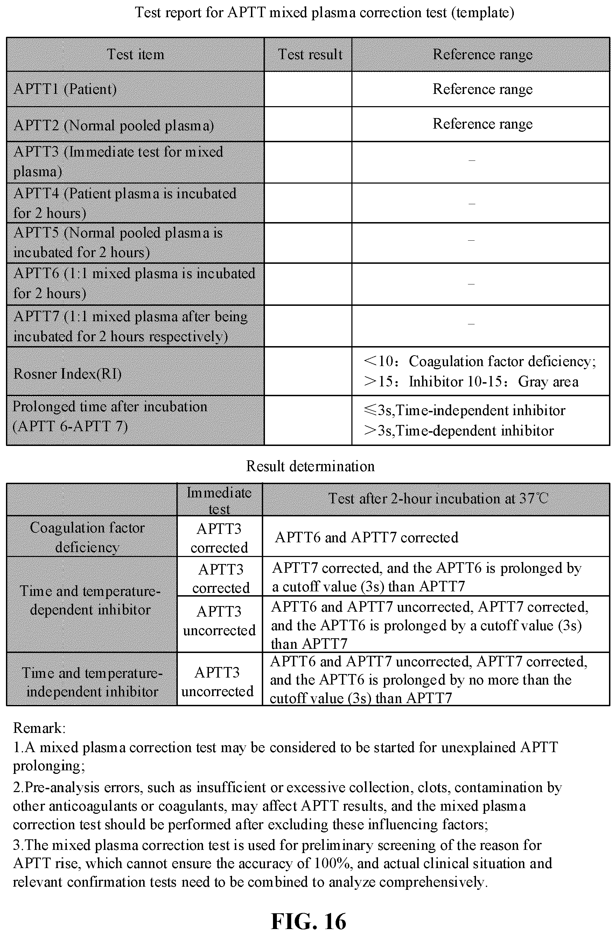

In some embodiments, the correction test report template may include: a name, a test result item, and a reference range item of a first coagulation item; a name, a test result item, and a reference range item of a second coagulation item; a name, a test result item, and a reference range item of a third coagulation item; a name, a test result item, and a reference range item of a fourth coagulation item; a name, a test result item, and a reference range item of a fifth coagulation item; a name, a test result item, and a reference range item of a sixth coagulation item; and a name, a test result item, and a reference range item of a seventh coagulation item. Reference is made to FIG. 16, which illustrates an example of a correction test report template.

In some embodiments, a result report may include: a name, a test result, and a reference range of a first coagulation item; a name, a test result, and a reference range of a second coagulation item; a name, a test result, and a reference range of a third coagulation item; a name, a test result, and a reference range of a fourth coagulation item; a name, a test result, and a reference range of a fifth coagulation item; a name, a test result, and a reference range of a sixth coagulation item; a name, a test result, and a reference range of a seventh coagulation item; a name of a calculation formula, a name of a calculation result, or the calculation formula itself; and the calculation result and/or the diagnosis result of the calculation formula.

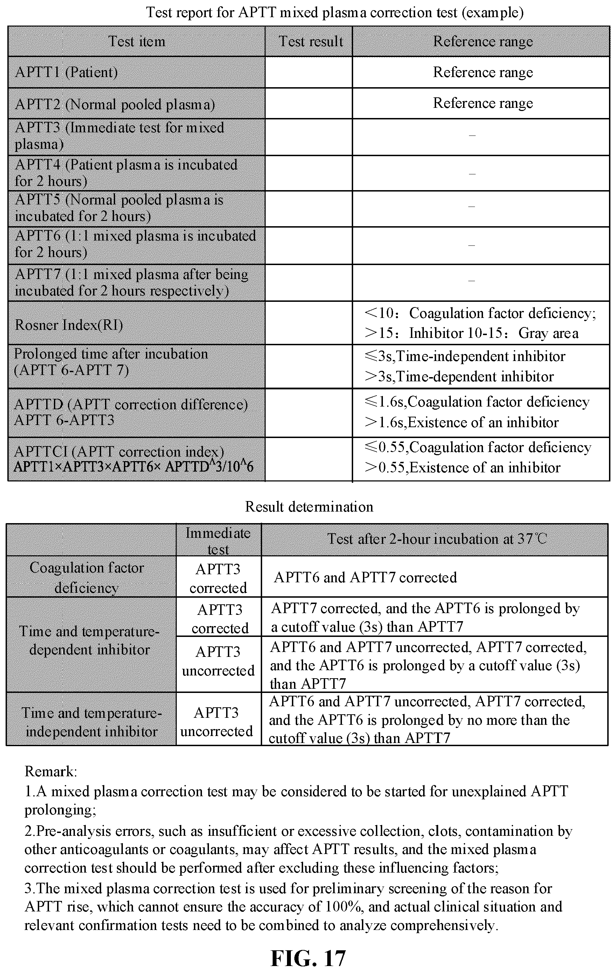

In some embodiments, a user may edit the result report through the input component, for example, the user can add self-defined parameter content to the result report. In some embodiments, each new parameter (calculation result) needs to contain the following information: the name of the parameter; the calculation formula of the parameter, herein a default parameter does not output the calculation formula, but a user-defined parameter will output the calculation formula, which is displayed in a remark area at the lower half part; the result of the parameter; the reference range of the parameter, herein most of the new parameters have no reference range, and will be replaced by "-"; and a description of a clinical significance of the parameter, which is allowed to be null. Reference is made to FIG. 17, which illustrates an example of a result report.

In some embodiments, a tool for correction test and clinical study is provided by allowing a user to define a calculation formula. The tool includes an operation interface and database support, is convenient for a medical worker to carry out the correction test and clinical study, and improves the screening and diagnostic capacity of the correction test.

The above is an example of obtaining a reason for the prolonged coagulation time by analyzing and calculating the test results through formulas.

In some embodiments, the test results can also be analyzed by means of a curve and chart.

For example, taking the coagulation time or a prolongation rate of the coagulation time as a vertical axis and a mixing ratio or mixing rate of patient plasma to normal plasma as a horizontal axis, two curves are drawn for immediate correction (CT1, CT2, and CT3) and incubation correction (CT4, CT5, and CT6), respectively. CT1, CT2, CT3, CT4, CT5, and CT6 represent the first to sixth coagulation time respectively. In this way, each curve contains at least three points. The mixed plasma is formed by mixing patient plasma and normal plasma in at least one ratio, so as to identify a coagulation factor inhibitor with low titer. Here, it is recommended to select the mixing ratios of 1/4 and 1/1. The prolongation rate of the coagulation time refers to that: the difference value between the coagulation time of the patient plasma and the coagulation time of the normal plasma is set as 1, and the value obtained by dividing the difference value between the coagulation time of the mixed plasma prepared by mixing the patient plasma and the normal plasma in a certain ratio and the coagulation time of the normal plasma by the difference value between the coagulation time of the patient plasma and the coagulation time of the normal plasma is determined as a drawing value. When the mixing ratio or the mixing rate of the patient plasma to the normal plasma is used as the horizontal axis, the value of the plasma sample with the patient plasma only may be set as 1, and the value of the plasma sample with the normal plasma only may be set as 0, or vice versa.

Therefore, in some embodiments, the processor is configured to draw an immediate correction curve according to the test results of various testing samples (such as the test results of the first testing sample, the second testing sample, and the third testing sample) of the immediate correction test and draw an incubation correction curve according to the test results of various testing samples (such as the test results of the fourth testing sample, the fifth testing sample, and the sixth testing sample) of the incubation correction test, by taking a coordinate system with the mixing ratio or mixing rate of patient plasma to normal plasma as the horizontal axis and the prolongation rate of the coagulation time or a blood coagulation time as the vertical axis. The display 400 displays the immediate correction curve and the incubation correction curve in the abovementioned coordinate system.

In some embodiments, the processor 300 is further configured to calculate areas enclosed by the abovementioned immediate correction curve and incubation correction curve. It can be understood that it is necessary to connect the first point of the immediate correction curve (such as a point drawn for a first testing sample) with the first point of the incubation correction curve (such as a point drawn for a fourth testing sample) and connect the last point of the immediate correction curve (such as a point drawn for a third testing sample) with the last point of the incubation correction curve (such as a point drawn for the fourth testing sample), to calculate the area enclosed by the abovementioned immediate correction curve and incubation correction curve. It can be understood that the sequence of the points here is determined by the values of the abscissa of the points.

A set of standard area values can be determined in advance, and the abovementioned test and calculation processes are performed on the sample with positive time-dependent coagulation factor inhibitor (for example, a common FVIII coagulation factor inhibitor). The processor 300 is further configured to obtain the reason for the prolonged coagulation time of the patient plasma according to the abovementioned area and an area threshold (that is, the standard area value above) for the display 400 to display the reason; and/or, the display 400 is further configured to display the value of the abovementioned area and the abovementioned area threshold. In some embodiments, when it is determined that the area is less than the area threshold, the processor 300 determines that there is no time-dependent coagulation inhibitor. When it is determined that the area is greater than the area threshold, the processor 300 determines that there is a time-dependent coagulation inhibitor.

For the reason for prolonged coagulation time without a time-dependent coagulation inhibitor (deficiency of a coagulation factor, coagulation factor inhibitors except FVIII, most LA), the conclusions of the immediate correction test and the incubation correction test are basically consistent no matter they are corrected or not. For the reason for prolonged coagulation time with a time-dependent coagulation inhibitor (FVIII coagulation factor inhibitor, a small amount of LA), the immediate correction test generally shows that it can be corrected or partially corrected, and the incubation correction test generally shows that it cannot be corrected.

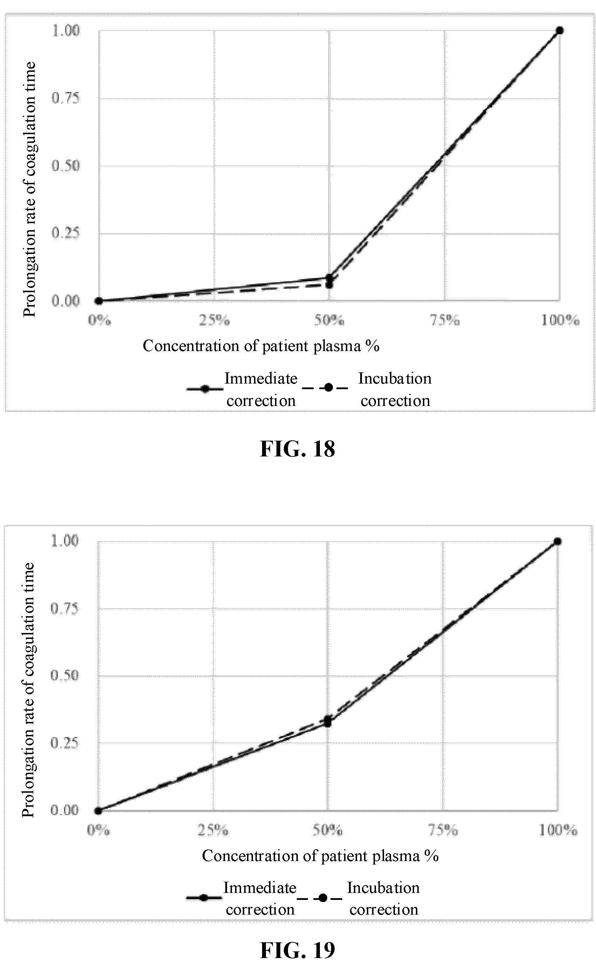

FIG. 18 and FIG. 19 show two reasons for the prolonged coagulation time without a time-dependent coagulation inhibitor, i.e., coagulation factor deficiency (hereditary hemophilia) and LA, and the area enclosed by the immediate correction curve and incubation correction curve is very small. For example, in FIG. 18, the immediate correction curve and the incubation correction curve basically coincide, and the area enclosed by the two curves is only 0.0125. In FIG. 19, it can be seen that the immediate correction curve and the incubation correction curve basically coincide, and the area enclosed by the two curves is only 0.0090. It is to be noted that the APTT reagent of Mindray is used here, and the LAC of the sample is 2.76, which is strongly positive for LA.

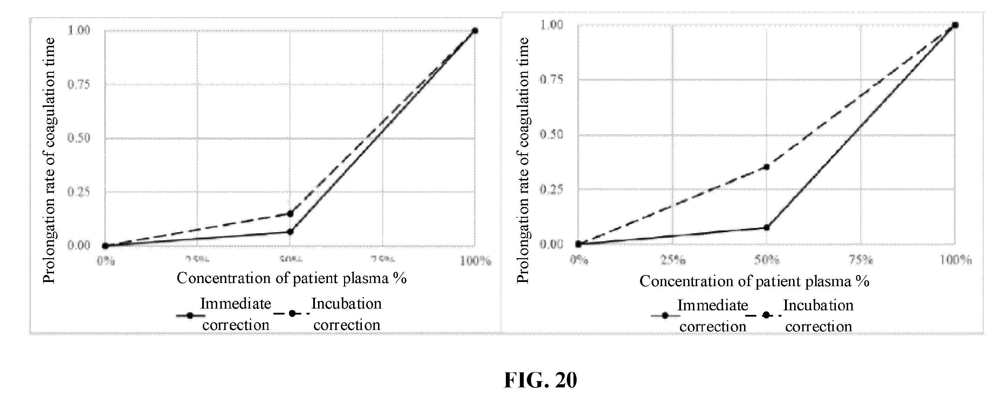

FIG. 20 shows a reason for the prolonged coagulation time with a time-dependent coagulation inhibitor, i.e., containing FVIII coagulation factor inhibitor (acquired hemophilia). The area enclosed by the immediate correction curve and incubation correction curve is relatively large, and with the increase of titer, the enclosed area increases. Specifically, FIG. 20 shows two drawings results for two patients with acquired hemophilia (the titer in the first figure, i.e., a lower figure, is about 2 BU/mL, and the titer in the second figure, i.e., an upper figure, is about 5 BU/mL). It can be seen that the area enclosed by the immediate correction curve and the incubation correction curve has increased to a certain extent, because the patient plasma contains a time-dependent FVIII coagulation factor inhibitor. The size of the enclosed area can characterize the titer of the coagulation factor inhibitor.

In FIG. 18 to FIG. 20, the curve represented by a dotted line is an incubation correction curve, and a curve represented by a solid line is an immediate correction curve. The concentration of patient plasma herein is the mixing ratio or mixing rate of patient plasma or normal plasma.

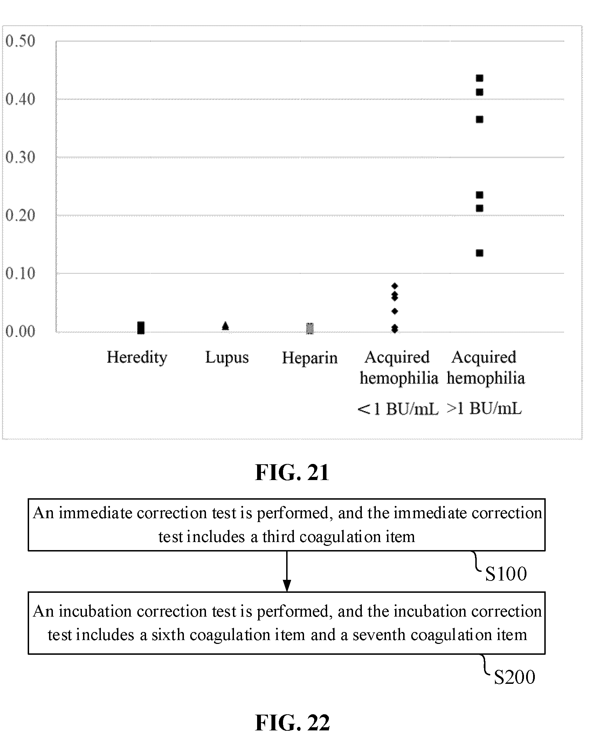

In some embodiments, the area threshold of the area enclosed by the immediate correction curve and the incubation correction curve refers to a detection limit that can identify the time-dependent coagulation factor inhibitor, which corresponds to coagulation factor deficiency and LA positive. As shown in FIG. 21, it can be seen from the figure that the enclosed areas corresponding to heparin, LA positive, hereditary hemophilia, etc. are generally below 0.08, which can be used as the area threshold of the test system. When the FVIII coagulation factor inhibitor is at a low titer (<1 BU/mL), it can hardly be distinguished from the reason of the prolonged coagulation time caused by the time-independent coagulation inhibitor. It can be easily distinguished only after a certain titer is reached. The area threshold is closely related to a reagent component and test methodology. Each test system should establish its own area threshold independently. However, it may draw only to just identity the existence of the time-dependence coagulation inhibitor without making a decision.

In some embodiments, the processor 300 is further configured to draw at least four points according to the test results of various testing samples (such as the test results of the fourth testing sample to the seventh testing sample) of the incubation correction test by taking a coordinate system with the mixing ratio or mixing rate of patient plasma or normal plasma as the horizontal axis and the prolongation rate of a coagulation time or a blood coagulation time as the vertical axis, and connect the at least four points in sequence to form a polygon. The processor 300 also calculates the area of the polygon. The processor 300 is further configured to obtain the reason for the prolonged coagulation time of the patient plasma according to the area of the polygon and an area threshold for the display 400 to display the reason; and/or, the display is further configured to display the value of the area of the polygon and the area threshold. In some embodiments, when it is determined that the area of the polygon is less than the area threshold, the processor 300 determines that there is no time-dependent coagulation inhibitor. When it is determined that the area of the polygon is greater than the area threshold, the processor 300 determines that there is a time-dependent coagulation inhibitor.

In some embodiments, the coagulation inhibitor includes a coagulation factor inhibitor and LA.

A sample analysis method is also provided in some embodiments of the invention. Referring to FIG. 22, the sample analysis method in some embodiments includes the following operations.

At S100, an immediate correction test is performed. In an embodiment, the immediate correction test performed at S100 includes the following operations. A testing sample for the immediate correction test is prepared by mixing a plasma sample for the immediate correction test with a reagent for determining a coagulation time. The testing sample for the immediate correction test is detected to obtain the coagulation time. In some embodiments, for the immediate correction test, a sample is aspirated from a sample vessel and is dispensed into a reaction vessel at S100, so as to prepare the testing sample for the immediate correction test in the reaction vessel.

At S200, an incubation correction test is performed. In an embodiment, the incubation correction test performed at S200 includes the following operations. A testing sample for the incubation correction test is prepared by mixing a plasma sample for the incubation correction test with a reagent for determining a coagulation time. The testing sample for the incubation correction test is detected to obtain the coagulation time. In some embodiments, for the incubation correction test, a sample is aspirated from the sample vessel and is dispensed into a buffer vessel to incubate for a preset duration under a preset condition at S200, and then the incubated sample is aspirated from the buffer vessel and is dispensed into the reaction vessel to prepare the testing sample for the incubation correction test in the reaction vessel.

In some embodiments, an initial amount of the plasma sample for the incubation correction test at the beginning of incubation is greater than a amount of the plasma sample for the immediate correction test.

The immediate correction test and the incubation correction test are described in more detail below.

In some embodiments, the immediate correction test at least includes a third coagulation item. In some embodiments, the immediate correction test further includes a first coagulation item and/or a second coagulation item, which will be specifically described below.

In the first coagulation item, the plasma sample for the immediate correction test is patient plasma. At S100, the patient plasma and a reagent for determining a coagulation time are mixed to prepare a first testing sample. In a specific embodiment, when the first testing sample is prepared at S100, a sample is aspirated from a sample vessel containing the patient plasma, a first amount of sample is dispensed into a first reaction vessel, and the reagent for determining the coagulation time is added to the first reaction vessel to prepare the first testing sample. The first testing sample is detected to obtain a first coagulation time.

In the second coagulation item, the plasma sample for the immediate correction test is normal plasma. At S100, the normal plasma and a reagent for determining a coagulation time are mixed to prepare a second testing sample. In a specific embodiment, when the second testing sample is prepared at S100, a sample is aspirated from a sample vessel containing the normal plasma, a second amount of sample is dispensed into a second reaction vessel, and the reagent for determining the coagulation time is added to the second reaction vessel to prepare the second testing sample. The second testing sample is detected to obtain a second coagulation time.

The immediate correction test at least includes a third coagulation item. In the third coagulation item, the plasma sample for the immediate correction test is mixed plasma formed by mixing the patient plasma and the normal plasma. At S100, the mixed plasma formed by mixing the patient plasma and the normal plasma is mixed with a reagent for determining a coagulation time to prepare a third testing sample. In a specific embodiment, when the third testing sample is prepared at S100, samples are respectively aspirated from the sample vessel containing the patient plasma and the sample vessel containing the normal plasma, the samples are dispensed into a third reaction vessel, the total amount of the samples dispensed into the third reaction vessel is a third amount, and the reagent for determining the coagulation time is added to the third reaction vessel to prepare the third testing sample. The third testing sample is detected to obtain a third coagulation time.

In some embodiments, the incubation correction test at least includes a sixth coagulation item and a seventh coagulation item. In some embodiments, the incubation correction test further includes a fourth coagulation item and/or a fifth coagulation item, which will be specifically described below.

In the fourth coagulation item, the plasma sample for the incubation correction test is the patient plasma incubated for a preset duration under a preset condition. At S200, the patient plasma is incubated for a preset duration under a preset condition, and the incubated patient plasma and a reagent for determining a coagulation time are mixed to prepare a fourth testing sample. In a specific embodiment, when the fourth testing sample is prepared at S200, the sample is aspirated from the sample vessel containing the patient plasma, a fourth amount of the sample is dispensed into a fourth buffer vessel, the sample is aspirated from the fourth buffer vessel after being incubated for a preset duration under a preset condition and is dispensed into a fourth reaction vessel, and the reagent for determining the coagulation time is added into the fourth reaction vessel to prepare the fourth testing sample. The fourth testing sample is detected to obtain a fourth coagulation time.

In the fifth coagulation item, the plasma sample for the incubation correction test is normal plasma incubated for a preset duration under a preset condition. At S200, the normal plasma is incubated for a preset duration under a preset condition, and the incubated normal plasma and a reagent for determining a coagulation time are mixed to prepare a fifth testing sample. In a specific embodiment, when the fifth testing sample is prepared at S200, the sample is aspirated from the sample vessel containing the normal sample, a fifth amount of sample is dispensed into a fifth buffer vessel, the sample is aspirated from the fifth buffer vessel after being incubated for a preset duration under a preset condition and is dispensed into a fifth reaction vessel, and the reagent for determining the coagulation time is added into the fifth reaction vessel to prepare the fifth testing sample. The fifth testing sample is detected to obtain a fifth coagulation time.

In the sixth coagulation item, the plasma sample for the incubation correction test is mixed plasma formed by mixing patient plasma and normal plasma in at least one ratio. At S200, a mixed sample formed by the patient plasma and the normal plasma is incubated for a preset duration under a preset condition, and the incubated mixed sample and a reagent for determining a coagulation time are mixed to prepare a sixth testing sample. In a specific embodiment, at S200, samples are respectively aspirated from the sample vessel containing the patient sample and the sample vessel containing the normal sample, the samples are dispensed into a sixth buffer vessel, the total amount of the samples dispensed into the six buffer vessel is a sixth amount, the sample is aspirated from the sixth buffer vessel after being incubated for a preset duration under a preset condition and is dispensed into a sixth reaction vessel, and the reagent for determining the coagulation time is added into the sixth reaction vessel to prepare the sixth testing sample. The sixth testing sample is detected to obtain a sixth coagulation time.

In the seventh coagulation item, the plasma sample for the incubation correction test is a plasma sample formed by mixing patient plasma incubated for a preset duration under a preset condition and normal plasma incubated for a preset duration under a preset condition. At S200, each of the patient plasma and the normal plasma are incubated for a preset duration under a preset condition, the incubated patient plasma, the incubated normal plasma, and the reagent for determining the coagulation time are mixed to prepare a seventh testing sample. In a specific embodiment, at S200, the incubated samples are respectively aspirated from the fourth buffer vessel and the fifth buffer vessel and dispensed into a seventh reaction vessel, and the reagent for determining the coagulation time is added into the seventh reaction vessel to prepare the seventh testing sample. The seventh testing sample is detected to obtain a seventh coagulation time.

The invention is described with reference to various exemplary embodiments. However, those skilled in the art will recognize that changes and modifications may be made to the exemplary embodiments without departing from the scope of the invention. For example, various operation steps and the components arranged to perform the operation steps can be implemented in different modes according to the specific application or considering any number of cost functions associated with the operation of the system (for example, one or more steps can be deleted, modified or combined into other steps).

The abovementioned embodiments may be implemented completely or partially through software, hardware, firmware, or any combination thereof. In addition, as understood by those skilled in the art, the principle of the invention can be reflected in a computer program product on a computer-readable storage medium. The computer-readable storage medium is per-installed with computer-readable program codes. Any tangible and non-transitory computer-readable storage medium can be used, including magnetic storage devices (hard disk, floppy disk, etc.), optical storage devices (CD to ROM, DVD, Blu Ray disk, etc.), flash memory, and/or the like. These computer program instructions may be loaded to a general-purpose computer, a special-purpose computer, or other programmable data processing devices to form a machine, such that these instructions executed on the computer or other programmable data processing devices can generate an apparatus for realizing a specified function. These computer program instructions may also be stored in a computer-readable memory. The computer-readable memory may instruct the computer or other programmable data processing devices to run in a specified mode. Thus, the instructions stored in the computer-readable memory may form a manufactured product, including an apparatus for realizing a specified function. These computer program instructions may also be loaded onto a computer or other programmable data processing devices, such that a series of operating steps are performed on the computer or other programmable data processing devices to produce a computer-implemented process. Therefore, instructions executed on the computer or other programmable data processing devices can provide steps for realizing the specified functions.

Although the principles herein have been shown in various embodiments, many modifications of structures, arrangements, proportions, elements, materials, and components that are particularly applicable to specific environmental and operational requirements may be used without departing from the principle and scope of the invention.

**[0147]** [00236] The foregoing specific description has been described with reference to various embodiments. However, those skilled in the art will recognize that various changes and modifications may be made without departing from the scope of the invention. Accordingly, the description of the invention will be illustrative rather than restrictive. Similarly, the advantages, other advantages, and solutions to problems of the various embodiments have been described above. However, the benefits, advantages, solutions to problems and any solution that can produce these elements or make them more explicit should not be interpreted as critical, essential, or necessary. As used herein, the term "include" and any other variations thereof refers to a non-exclusive inclusion, so that a process, method, article or device that includes a list of elements includes not only these elements, but also other elements that are not explicitly listed or do not belong to the process, method, system, article or device. Further, as used herein, the term "couple" and any other variations thereof refer to a physical connection, an electrical connection, a magnetic connection, an optical connection, a communication connection, a functional connection, and/or any other connection. Those skilled in the art will recognize that many changes can be made to the details of the abovementioned embodiments without departing from the basic principle of the invention. Therefore, the scope of the invention is limited only by the claims.

**Claims**

1. A sample analysis apparatus, comprising a testing sample preparation component (100), a detection component (200) and a processor (300) connected to the testing sample preparation component (100) and the detection component (200), wherein the testing sample preparation component (100) comprises a reaction vessel loading component (10), a sample feeding component (20), a sample dispensing component (30), a reagent carrying component (40), a reagent dispensing component (60), a reaction component (50) and a scheduling mechanism (70), wherein:

    the reagent carrying component (40) is configured to carry a reagent vessel, wherein the reagent vessel contains a reagent, and the reagent comprises a reagent for determining a coagulation time; and the processor (300) is configured to:

    control the reaction vessel loading component (10) to supply a reaction vessel and/or a buffer vessel;
    control the sample feeding component (20) to supply a sample vessel, wherein the sample vessel contains a sample, and the sample is patient plasma or normal plasma;
    control the sample dispensing component (30) to aspirate the sample in the sample vessel and dispense the sample into the reaction vessel or the buffer vessel;
    control the reagent dispensing component (60) to aspirate the reagent from the reagent vessel and dispense the reagent into the reaction vessel;
    control the reaction component (50) to incubate the reaction vessel and/or the buffer vessel carrying the sample, and to incubate the reaction vessel carrying the sample and the reagent;
    control the scheduling mechanism (70) to schedule the reaction vessel and the buffer vessel, to schedule the reaction vessel to be transferred to the reaction component (50) and schedule the buffer vessel to be transferred to the reaction component (50); and
    control the detection component (200), preferably an optical detection component, a double-magnetic bead method-based detection component, or an optical and magnetic integrated detection component, to detect a testing sample formed by mixing the sample and the reagent,

    wherein the processor (300) is further configured to:

    control the sample dispensing component (30) to aspirate the patient plasma from the sample vessel containing the patient plasma and the normal plasma from the sample vessel containing the normal plasma, and to dispense the patient plasma and the normal plasma into a third reaction vessel to form mixed plasma;
    control the reagent dispensing component (60) to aspirate the reagent for determining the coagulation time from the reagent vessel, and to add the reagent for determining the coagulation time into the third reaction vessel to prepare a third testing sample;
    control the sample dispensing component (30) to aspirate the patient plasma from the sample vessel containing the patient plasma and the normal plasma from the sample vessel containing the normal plasma, and to dispense the patient plasma and the normal plasma into a sixth buffer vessel to form mixed plasma;
    control the reaction component (50) to incubate the mixed plasma in the sixth buffer vessel for 0.5 to 4 hours at a temperature of 30 to 45°C; control the scheduling mechanism (70) to schedule the sixth buffer vessel to be transferred to the reaction component (50); control the sample dispensing component (30) to aspirate the incubated mixed plasma from the sixth buffer vessel and dispense the incubated mixed plasma into a sixth reaction vessel; and control the reagent dispensing component (60) to aspirate the reagent for determining the coagulation time from the reagent vessel, and to add the reagent for determining the coagulation time into the sixth reaction vessel to prepare a sixth testing sample;
    **characterized in that** the processor (300) is further configured to:

    control the sample dispensing component (30) to aspirate the patient plasma from the sample vessel containing the patient plasma and dispense the patient plasma into a fourth buffer vessel; control the reaction component (50) to incubate the patient plasma in the fourth buffer vessel for 0.5 to 4 hours at a temperature of 30 to 45°C; control the scheduling mechanism (70) to schedule the fourth buffer vessel to be transferred to the reaction component (50); control the sample dispensing component (30) to aspirate the normal plasma from the sample vessel containing the normal plasma and dispense the normal plasma into a fifth buffer vessel; control the reaction component (50) to incubate the normal plasma in the fifth buffer vessel for 0.5 to 4 hours at a temperature of 30 to 45°C; control the scheduling mechanism (70) to schedule the fifth buffer vessel to be transferred to the reaction component (50); control the sample dispensing component (30) to aspirate the incubated patient plasma from the fourth buffer vessel and the incubated normal plasma from the fifth buffer vessel, and dispense the incubated patient plasma and the incubated normal plasma into a seventh reaction vessel; and control the reagent dispensing component (60) to aspirate the reagent for determining the coagulation time from the reagent vessel, and to add the reagent for determining the coagulation time into the seventh reaction vessel to prepare a seventh testing sample; and

control the detection component (200) to detect the third testing sample to obtain a third coagulation time, detect the sixth testing sample to obtain a sixth coagulation time, and detect the seventh testing sample to obtain a seventh coagulation time.

2. The sample analysis apparatus of claim 1, wherein the processor (300) is further configured to:

control the sample dispensing component (30) to aspirate the incubated patient plasma from the fourth buffer vessel and dispense the incubated patient plasma into a fourth reaction vessel; control the reagent dispensing component (60) to aspirate the reagent for determining the coagulation time from the reagent vessel and add the reagent for determining the coagulation time into the fourth reaction vessel to prepare a fourth testing sample; control the sample dispensing component (30) to aspirate the incubated normal plasma from the fifth buffer vessel and dispense the incubated normal plasma into a fifth reaction vessel; control the reagent dispensing component (60) to aspirate the reagent for determining the coagulation time from the reagent vessel and add the reagent for determining the coagulation time into the fifth reaction vessel to prepare a fifth testing sample; and control the detection component (200) to detect the fourth testing sample to obtain a fourth coagulation time, and detect the fifth testing sample to obtain a fifth coagulation time.

3. The sample analysis apparatus of claim 1 or 2, wherein the processor (300) is further configured to:

after the sample dispensing component (30) aspirates the incubated patient plasma from the fourth buffer vessel, control the reaction component (50) to continue to incubate the patient plasma in the fourth buffer vessel at a temperature of 30 to 45°C, to enable the patient plasma in the fourth buffer vessel to be used for retesting; and/or, after the sample dispensing component (30) aspirates the incubated normal plasma from the fifth buffer vessel, control the reaction component (50) to continue to incubate the normal plasma in the fifth buffer vessel at a temperature of 30 to 45°C, to enable the normal plasma in the fifth buffer vessel to be used for retesting; and/or, after the sample dispensing component (30) aspirates the incubated mixed plasma from the sixth buffer vessel, control the reaction component (50) to continue to incubate the mixed plasma in the sixth buffer vessel at a temperature of 30 to 45°C, to enable the mixed plasma in the sixth buffer vessel to be used for retesting.

4. The sample analysis apparatus of claim 1, wherein an amount of the mixed plasma dispensed into the third reaction vessel by the sample dispensing component (30) is a third amount, an amount of the mixed plasma dispensed into the sixth buffer vessel by the sample dispensing component (30) is a sixth amount, and a total amount of the patient plasma dispensed into the fourth buffer vessel and the normal plasma dispensed into the fifth buffer vessel by the sample dispensing component (30) is a seventh amount; and wherein the seventh amount is greater than the third amount, and the sixth amount is greater than the third amount, preferably, the seventh amount is five to six times the third amount, and the sixth amount is five to six times the third amount.

5. The sample analysis apparatus of claim 1, further comprising a water replenishing apparatus (500), and the processor (300) is further configured to control the water replenishing apparatus (500) to dispense water into at least one of the fourth buffer vessel, the fifth buffer vessel, and the sixth buffer vessel during incubation according to a preset time interval and a preset amount for replenishing water.

6. The sample analysis apparatus of claim 1, wherein a depth-diameter ratio of the fourth buffer vessel is greater than a depth-diameter ratio threshold, a depth-diameter ratio of the fifth buffer vessel is greater than the depth-diameter ratio threshold, and a depth-diameter ratio of the sixth buffer vessel is greater than the depth-diameter ratio threshold; and wherein the depth-diameter ratio threshold is greater than 5.

7. The sample analysis apparatus of claim 1, wherein all of the fourth buffer vessel, the fifth buffer vessel, the sixth buffer vessel, the third reaction vessel, the sixth reaction vessel, and the seventh reaction vessel are of same structure, so that the fourth buffer vessel, the fifth buffer vessel, and the sixth buffer vessel are capable of being scheduled to be transferred into the reaction component (50) for incubating.

8. A sample analysis apparatus, comprising:

a testing sample preparation component (100), a detection component (200) and a processor (300), wherein the processor (300) is configured to control the testing sample preparation component (100) to:

aspirate patient plasma from a sample vessel containing the patient plasma and normal plasma from a sample vessel containing the normal plasma respectively, dispense the patient plasma and the normal plasma into a third reaction vessel to form mixed plasma, and add a reagent for determining a coagulation time into the third reaction vessel to prepare a third testing sample;

aspirate the patient plasma from the sample vessel containing the patient plasma and the normal plasma from the sample vessel containing the normal plasma respectively, dispense the patient plasma and the normal plasma into a sixth buffer vessel to form mixed plasma, incubate the mixed plasma in the sixth buffer vessel for 0.5 to 4 hours at a temperature of 30 to 45°C, aspirate the incubated mixed plasma from the sixth buffer vessel and dispense the incubated mixed plasma into a sixth reaction vessel, and add the reagent for determining the coagulation time into the sixth reaction vessel to prepare a sixth testing sample;

**characterized in that** the processor (300) is further configured to:

control the testing sample preparation component (100) to aspirate the patient plasma from the sample vessel containing the patient plasma and dispense the patient plasma into a fourth buffer vessel, incubate the patient plasma in the fourth buffer vessel for 0.5 to 4 hours at a temperature of 30 to 45°C, aspirate the normal plasma from the sample vessel containing the normal plasma and dispense the normal plasma into a fifth buffer vessel, incubate the normal plasma in the fifth buffer vessel for 0.5 to 4 hours at a temperature of 30 to 45°C; aspirate the incubated patient plasma from the fourth buffer vessel and the incubated normal plasma from the fifth buffer vessel respectively and dispense the incubated patient plasma and the incubated normal plasma into a seventh reaction vessel, and add the reagent for determining the coagulation time into the seventh reaction vessel to prepare a seventh testing sample; and

control the detection component (200), preferably an optical detection component, a double-magnetic bead method-based detection component, or an optical and magnetic integrated detection component, to detect the third testing sample to obtain a third coagulation time, detect the sixth testing sample to obtain a sixth coagulation time, and detect the seventh testing sample to obtain a seventh coagulation time.

9. The sample analysis apparatus of claim 8, wherein the processor (300) is further configured to control the testing sample preparation component (100) to: aspirate the incubated patient plasma from the fourth buffer vessel and dispense the incubated patient plasma into a fourth reaction vessel, and add the reagent for determining the coagulation time into the fourth reaction vessel to prepare a fourth testing sample; and the processor (300) is further configured to control the testing sample preparation component (100) to: aspirate the incubated normal plasma from the fifth buffer vessel and dispense the incubated normal plasma into a fifth reaction vessel, and add the reagent for determining the coagulation time into the fifth reaction vessel to prepare a fifth testing sample; and

the processor (300) is further configured to control the detection component (200) to detect the fourth testing sample to obtain a fourth coagulation time, and detect the fifth testing sample to obtain a fifth coagulation time.

10. The sample analysis apparatus of claim 8, wherein the processor (300) is further configured to:

after aspirating the incubated patient plasma from the fourth buffer vessel, control the testing sample preparation component (100) to continue to incubate the patient plasma in the fourth buffer vessel at a temperature of 30 to 45°C, to enable the patient plasma in the fourth buffer vessel to be used for retesting; and/or,

after aspirating the incubated normal plasma from the fifth buffer vessel, control the testing sample preparation component (100) to continue to incubate the normal plasma in the fifth buffer vessel at a temperature of 30 to 45°C, to enable the normal plasma in the fifth buffer vessel to be used for retesting; and/or,

after aspirating the incubated mixed plasma from the sixth buffer vessel, control the testing sample preparation component (100) to continue to incubate the mixed plasma in the sixth buffer vessel at a temperature of 30 to 45°C, to enable the mixed plasma in the sixth buffer vessel to be used for retesting.

11. The sample analysis apparatus of claim 8, wherein an amount of the mixed plasma dispensed into the third reaction vessel by the testing sample preparation component (100) is a third amount, an amount of the mixed plasma dispensed into the sixth buffer vessel by the testing sample preparation component (100) is a sixth amount, and a total amount of the patient plasma dispensed into the fourth buffer vessel and the normal plasma dispensed into the fifth buffer vessel by the testing sample preparation component (100) is a seventh amount; and wherein the seventh amount is greater than the third amount, and the sixth amount is greater than the third amount, preferably, the seventh amount is five to six times the third amount, and the sixth amount is five to six times the third amount.

12. The sample analysis apparatus of claim 8, further comprising a water replenishing apparatus (500), wherein the processor (300) is further configured to control the water replenishing apparatus (500) to dispense water into at least one of the fourth buffer vessel, the fifth buffer vessel, and the sixth buffer vessel during incubation according to a preset time interval and a preset amount for replenishing water.

13. The sample analysis apparatus of claim 8, wherein a depth-diameter ratio of the fourth buffer vessel is greater than a depth-diameter ratio threshold, a depth-diameter ratio of the fifth buffer vessel is greater than the depth-diameter ratio threshold, and a depth-diameter ratio of the sixth buffer vessel is greater than the depth-diameter ratio threshold; and wherein the depth-diameter ratio threshold is greater than 5.

14. The sample analysis apparatus of claim 8, wherein all of the fourth buffer vessel, the fifth buffer vessel, the sixth buffer vessel, the third reaction vessel, the sixth reaction vessel, and the seventh reaction vessel are of same structure, so that the fourth buffer vessel, the fifth buffer vessel, and the sixth buffer vessel are capable of being scheduled to be transferred into the testing sample preparation component for incubating.

15. A sample analysis method, comprising:

performing (S100) an immediate correction test, wherein the immediate correction test comprises:

preparing a testing sample for the immediate correction test, wherein the testing sample for the immediate correction test is formed by mixing a sample for the immediate correction test and a reagent for determining a coagulation time; and
detecting the testing sample for the immediate correction test to obtain a coagulation time;

performing (S200) an incubation correction test, wherein the incubation correction test comprises:

preparing a testing sample for the incubation correction test, wherein the testing sample for the incubation correction test is formed by mixing a sample for the incubation correction test and the reagent for determining the coagulation time; and
detecting the testing sample for the incubation correction test to obtain a coagulation time;

wherein
the immediate correction test at least comprises a third coagulation item; in the third coagulation item, the sample for the immediate correction test is mixed plasma formed by mixing patient plasma and normal plasma, and the third coagulation item comprises:

acquiring the patient plasma and the normal plasma, mixing the patient plasma and the normal plasma to form the mixed plasma, and mixing the mixed plasma and the reagent for determining the coagulation time to prepare a third testing sample;
detecting the third testing sample to obtain a third coagulation time;
wherein
the incubation correction test at least comprises a sixth coagulation item and a seventh coagulation item;
in the sixth coagulation item, the sample for the incubation correction test is mixed plasma formed by mixing the patient plasma and the normal plasma, and the sixth coagulation item comprises:

acquiring the patient plasma and the normal plasma, mixing the patient plasma and the normal plasma to form the mixed plasma, incubating the mixed plasma for a preset duration under a preset condition, and mixing the incubated mixed plasma and the reagent for determining the coagulation time to prepare a sixth testing sample;
detecting the sixth testing sample to obtain a sixth coagulation time;
**characterized in that**
in the seventh coagulation item, the sample for the incubation correction test is mixed plasma formed by mixing the patient plasma and the normal plasma, and the seventh coagulation item comprises:

acquiring the patient plasma and the normal plasma, incubating each of the patient plasma and the normal plasma for a preset duration under a preset condition, mixing the incubated patient plasma and the incubated normal plasma to form the mixed plasma, and mixing the mixed plasma formed by mixing the incubated patient plasma and the incubated normal plasma and the reagent for determining the coagulation time to prepare a seventh testing sample; and

detecting the seventh testing sample to obtain a seventh coagulation time.

**Patentansprüche**

1. Eine Probenanalysevorrichtung, die eine Komponente zur Vorbereitung von Testproben (100), eine Nachweiskomponente (200) und einen Prozessor (300) umfasst, der mit der Komponente zur Vorbereitung von Testproben (100) und der Nachweiskomponente (200) verbunden ist, wobei die Komponente zur Vorbereitung von Testproben (100) eine Reaktionsgefäß- Befüllkomponente (10), eine Probenzuführeinheit (20), eine Probenverteilungseinheit (30), eine Reagenzträgereinheit (40), eine Reagenzverteilungseinheit (60), eine Reaktionskomponente (50) und einen Ablaufsteuerungsmechanismus (70) umfasst, wobei:

die Reagenzträgereinheit (40) dazu konfiguriert ist, ein Reagenzgefäß, das ein Reagenz enthält, zu tragen. Bei dem Reagenz handelt es sich um ein Reagenz zur Bestimmung der Gerinnungszeit; und der Prozessor (300) ist dazu konfiguriert:

Steuerung der Reaktionsgefäß-Befüllkomponente (10) zur Bereitstellung eines Reaktionsgefäßes und/oder eines Puffergefäßes;

Steuerung der Probenzuführeinheit (20) zur Bereitstellung eines Probengefäßes, das eine Probe enthält, und es sich bei der Probe um Patientenplasma oder normales Plasma handelt;

Steuerung der Probenverteilungseinheit (30), um die Probe aus dem Probengefäß zu aspirieren und in das Reaktionsgefäß oder das Puffergefäß zu verabreichen;

Steuerung der Reagenzverteilungseinheit (60), um das Reagenz aus dem Reagenzgefäß zu aspirieren und in das Reaktionsgefäß zu verabreichen;

Steuerung der Reaktionskomponente (50), um das Reaktionsgefäß und/oder das Puffergefäß mit der Probe zu inkubieren sowie das Reaktionsgefäß mit der Probe und dem Reagenz zu inkubieren;

Steuerung des Ablaufsteuerungsmechanismus (70), um das Reaktionsgefäß und das Puffergefäß einzuplanen, das Reaktionsgefäß zur Überführung zur Reaktionskomponente (50) einzuplanen und das Puffergefäß zur Überführung zur Reaktionskomponente (50) einzuplanen; und

Steuerung der Nachweiskomponente (200), vorzugsweise einer optischen Nachweiskomponente, einer auf dem Doppel-Magnetperlenverfahren basierenden Nachweiskomponente oder einer integrierten optisch-magnetischen Nachweiskomponente, um eine Testprobe zu erfassen, die durch Mischen der Probe mit dem Reagenz gebildet wurde,

wobei der Prozessor (300) ferner dazu konfiguriert ist:

Steuerung der Probenverteilungseinheit (30), damit Patientenplasma aus dem Probengefäß mit dem Patientenplasma und normales Plasma aus dem Probengefäß mit dem normalen Plasma aspiriert und sowohl das Patientenplasma als auch das normale Plasma in ein drittes Reaktionsgefäß verabreicht werden, um gemischtes Plasma zu bilden; Steuerung der Reagenzverteilungseinheit (60), damit das Reagenz zur Bestimmung der Gerinnungszeit aus dem Reagenzgefäß aspiriert und in das dritte Reaktionsgefäß gegeben wird, um eine dritte Testprobe herzustellen;

Steuerung der Probenverteilungseinheit (30), damit das Patientenplasma aus dem Probengefäß mit dem Patientenplasma und normales Plasma aus dem Probengefäß mit dem normalen Plasma aspiriert und sowohl das Patientenplasma als auch das normale Plasma in ein sechstes Puffergefäß verabreicht werden, um gemischtes Plasma zu bilden;

Steuerung der Reaktionskomponente (50), damit das gemischte Plasma im sechsten Puffergefäß für 0,5 bis 4 Stunden bei einer Temperatur von 30 bis 45 °C inkubiert wird; Steuerung des Ablaufsteuerungsmechanismus (70), damit das sechste Puffergefäß zur Reaktionskomponente (50) überführt wird; Steuerung der Probenverteilungseinheit (30) so zu steuern, dass das inkubierte gemischte Plasma aus dem sechsten Puffergefäß aspiriert und in ein sechstes Reaktionsgefäß verbreicht wird; und Steuerung der Reagenzverteilungseinheit (60), damit das Reagenz zur Bestimmung der Gerinnungszeit aus dem Reagenzgefäß aspiriert und in das sechste Reaktionsgefäß gegeben wird, um eine sechste Testprobe herzustellen mit der Eigenschaft,

dass der Prozessor (300) weiterhin wie folgt konfiguriert ist:

Steuerung der Probenverteilungseinheit (30), damit Patientenplasma aus dem Probengefäß mit dem Patientenplasma aspiriert und in ein viertes Puffergefäß verabreicht wird; Steuerung der Reaktionskomponente (50), damit das Patientenplasma im vierten Puffergefäß für 0,5 bis 4 Stunden bei einer Temperatur von 30 bis 45 °C inkubiert wird; Steuerung des Ablaufsteuerungsmechanismus (70), damit das vierte Puffergefäß zur Reaktionskomponente (50) überführt wird; Steuerung der Probenverteilungseinheit (30), damit normales Plasma aus dem Probengefäß mit dem normalen Plasma aspiriert und in ein fünftes Puffergefäß verabreicht wird; Steuerung der Reaktionskomponente (50), damit das normale Plasma im fünften Puffergefäß für 0,5 bis 4 Stunden bei einer Temperatur von 30 bis 45 °C inkubiert wird; Steuerung des Ablaufsteuerungsmechanismus (70), damit das fünfte Puffergefäß zur Reaktionskomponente (50) überführt wird; Steuerung der Probenverteilungseinheit (30), damit das inkubierte Patientenplasma aus dem vierten Puffergefäß und das inkubierte normale Plasma aus dem fünften Puffergefäß aspiriert und sowohl das inkubierte Patientenplasma als auch das inkubierte normale Plasma in ein siebtes Reaktionsgefäß dosiert wird; Steuerung der Reagenzverteilungseinheit (60), damit das Reagenz zur Bestimmung der Gerinnungszeit aus dem Reagenzgefäß aspiriert und in das siebte Reaktionsgefäß gegeben wird, um eine siebte Testprobe herzustellen; und

Steuerung der Nachweiskomponente (200) zur Erfassung der dritten Testprobe, um eine dritte Gerinnungszeit zu erhalten, zur Erfassung der sechsten Testprobe, um eine sechste Gerinnungszeit zu erhalten, und Erfassung der siebten Testprobe, um eine siebte Gerinnungszeit zu erhalten.

2. Probenanalysevorrichtung nach Anspruch 1, wobei der Prozessor (300) weiterhin wie folgt konfiguriert ist:

   Steuerung der Probenverteilungseinheit (30), damit das inkubierte Patientenplasma aus dem vierten Puffergefäß aspiriert und in ein viertes Reaktionsgefäß verabreicht wird; Steuerung der Reagenzverteilungseinheit (60), damit das Reagenz zur Bestimmung der Gerinnungszeit aus dem Reagenzgefäß aspiriert und in das vierte Reaktionsgefäß gegeben wird, um eine vierte Testprobe herzustellen;
   Steuerung der Probenverteilungseinheit (30), damit das inkubierte normale Plasma aus dem fünften Puffergefäß aspiriert und in ein fünftes Reaktionsgefäß verbreicht wird; Steuerung der Reagenzverteilungseinheit (60), damit das Reagenz zur Bestimmung der Gerinnungszeit aus dem Reagenzgefäß aspiriert und in das fünfte Reaktionsgefäß gegeben wird, um eine fünfte Testprobe herzustellen; und
   Steuerung der Nachweiskomponente (200) zur Erfassung der vierten Testprobe, um eine vierte Gerinnungszeit zu erhalten, und Erfassung der fünften Testprobe, um eine fünfte Gerinnungszeit zu erhalten.

3. Probenanalysevorrichtung nach Anspruch 1 oder 2, wobei der Prozessor (300) ferner wie folgt konfiguriert ist:

   nachdem die Probenverteilungseinheit (30) das inkubierte Patientenplasma aus dem vierten Puffergefäß aspiriert hat, die Reaktionskomponente (50) so zu steuern, dass das Patientenplasma im vierten Puffergefäß weiterhin bei einer Temperatur von 30 bis 45 °C inkubiert wird, sodass das Patientenplasma im vierten Puffergefäß für eine Nachprüfung verwendet werden kann; und/oder
   nachdem die Probenverteilungseinheit (30) das inkubierte normale Plasma aus dem fünften Puffergefäß aspiriert hat, die Reaktionskomponente (50) so zu steuern, dass das normale Plasma im fünften Puffergefäß weiterhin bei einer Temperatur von 30 bis 45 °C inkubiert wird, sodass das normale Plasma im fünften Puffergefäß für eine Nachprüfung verwendet werden kann; und/oder
   nachdem die Probenverteilungseinheit (30) das inkubierte Mischplasma aus dem sechsten Puffergefäß aspiriert hat, die Reaktionskomponente (50) so zu steuern, dass das Mischplasma im sechsten Puffergefäß weiterhin bei einer Temperatur von 30 bis 45 °C inkubiert wird, sodass das Mischplasma im sechsten Puffergefäß für eine Nachprüfung verwendet werden kann.

4. Probenanalysevorrichtung nach Anspruch 1, wobei eine Menge des von der Probenverteilungseinheit (30) in das dritte Reaktionsgefäß verabreichten Mischplasmas einer dritten Menge entspricht, eine Menge des in das sechste Puffergefäß verabreichten Mischplasmas einer sechsten Menge entspricht, und eine Gesamtmenge des in das vierte Puffergefäß verabreichten Patientenplasmas und des in das fünfte Puffergefäß verabreichten normalen Plasmas einer siebten Menge entspricht; und wobei die siebte Menge größer ist als die dritte Menge, und die sechste Menge größer ist als die dritte Menge, vorzugsweise ist die siebte Menge fünf- bis sechsmal so groß wie die dritte Menge, und die sechste Menge ist fünf- bis sechsmal so groß wie die dritte Menge

5. Das Probenanalysevorrichtung nach Anspruch 1 umfasst ferner eine Wassernachfüllvorrichtung (500), wobei der Prozessor (300) weiterhin konfiguriert ist, um die Wassernachfüllvorrichtung (500) so zu steuern, dass Wasser gemäß einem voreingestellten Zeitintervall und einer voreingestellten Wassermenge während der Inkubation in mindestens eines der folgenden Gefäße verabreicht wird: das vierte Puffergefäß, das fünfte Puffergefäß und das sechste Puffergefäß.

6. Die Probenanalysevorrichtung nach Anspruch 1, wobei ein Verhältnis von Tiefe zu Durchmesser des vierten Puffergefäßes größer ist als ein Schwellenwert für das Verhältnis von Tiefe zu Durchmesser, ein Verhältnis von Tiefe zu Durchmesser des fünften Puffergefäßes größer ist als der Schwellenwert, und ein Verhältnis von Tiefe zu Durchmesser des sechsten Puffergefäßes größer ist als der Schwellenwert; und wobei der Schwellenwert für das Verhältnis von Tiefe zu Durchmesser größer als 5 ist.

7. Probenanalysevorrichtung nach Anspruch 1, wobei das vierte, fünfte und das sechste Puffergefäß, das dritte, sechste und das siebte Reaktionsgefäß alle dieselbe Struktur aufweisen, sodass das vierte, fünfte und sechste Puffergefäß geplant in die Reaktionskomponente (50) zur Inkubation übertragen werden können.

8. Eine Probenanalysevorrichtung, die Folgendes umfasst:
eine Komponente zur Vorbereitung von Testproben (100), eine Nachweiskomponente (200) und einen Prozessor (300), wobei der Prozessor (300) so konfiguriert ist, dass er die Komponente zur Vorbereitung von Testproben (100) steuert, um:

Patientenplasma aus einem Probengefäß, das das Patientenplasma enthält, und normales Plasma aus einem Probengefäß, das normales Plasma enthält, jeweils zu aspirieren, das Patientenplasma und das normale Plasma in ein drittes Reaktionsgefäß zu verabreichen, um Mischplasma zu bilden, und ein Reagenz zur Bestimmung der Gerinnungszeit in das dritte Reaktionsgefäß zu geben, um eine dritte Testprobe herzustellen; das Patientenplasma aus dem Probengefäß mit Patientenplasma und das normale Plasma aus dem Probengefäß mit normalem Plasma jeweils zu aspirieren, das Patientenplasma und das normale Plasma in ein sechstes Puffergefäß zu verabreichen, um Mischplasma zu bilden, das Mischplasma im sechsten Puffergefäß 0,5 bis 4 Stunden bei einer Temperatur von 30 bis 45 °C zu inkubieren, das inkubierte Mischplasma aus dem sechsten Puffergefäß zu aspirieren und in ein sechstes Reaktionsgefäß zu verabreichen, und das Reagenz zur Bestimmung der Gerinnungszeit in das sechste Reaktionsgefäß zu geben, um eine sechste Testprobe herzustellen; mit der Eigenschaft, dass der Prozessor (300) ferner wie folgt konfiguriert ist; Steuerung der Komponente zur Vorbereitung von Testproben (100), damit Patientenplasma aus einem Probengefäß, das das Patientenplasma enthält, aspiriert und in ein viertes Puffergefäß verabreicht wird, das Patientenplasma im vierten Puffergefäß 0,5 bis 4 Stunden bei einer Temperatur von 30 bis 45 °C inkubiert wird, normales Plasma aus einem Probengefäß mit normalem Plasma aspiriert und in ein fünftes Puffergefäß verabreicht wird, das normale Plasma im fünften Puffergefäß 0,5 bis 4 Stunden bei einer Temperatur von 30 bis 45 °C inkubiert wird; das inkubierte Patientenplasma aus dem vierten Puffergefäß und das inkubierte normale Plasma aus dem fünften Puffergefäß jeweils aspiriert und gemeinsam in ein siebtes Reaktionsgefäß verabreicht werden, und das Reagenz zur Bestimmung der Gerinnungszeit in das siebte Reaktionsgefäß zugegeben wird, um eine siebte Testprobe herzustellen; und Steuerung der Nachweiskomponente (200), vorzugsweise einer optischen Nachweiskomponente, einer auf der Doppel-Magnetperlen-Methode basierenden Nachweiskomponente oder einer optisch und magnetisch integrierten Nachweiskomponente, um die dritte Testprobe zu analysieren und eine dritte Gerinnungszeit zu ermitteln, die sechste Testprobe zu analysieren und eine sechste Gerinnungszeit zu ermitteln, und die siebte Testprobe zu analysieren und eine siebte Gerinnungszeit zu ermitteln.

9. Die Probenanalysevorrichtung nach Anspruch 8, wobei der Prozessor (300) ferner konfiguriert ist, die Komponente zur Vorbereitung von Testproben (100) so zu steuern, dass das inkubierte Patientenplasma aus dem vierten Puffergefäß aspiriert und in ein viertes Reaktionsgefäß verabreicht wird, und das Reagenz zur Bestimmung der Gerinnungszeit in das vierte Reaktionsgefäß zugegeben wird, um eine vierte Testprobe herzustellen; und die Komponente zur Vorbereitung von Testproben (100) so zu steuern, dass das inkubierte normale Plasma aus dem fünften Puffergefäß aspiriert und in ein fünftes Reaktionsgefäß verabreicht wird, und das Reagenz zur Bestimmung der Gerinnungszeit in das fünfte Reaktionsgefäß zugegeben wird, um eine fünfte Testprobe herzustellen; und
der Prozessor (300) ist weiterhin konfiguriert, die Nachweiskomponente (200) zu steuern, um die vierte Testprobe zu analysieren und eine vierte Gerinnungszeit zu ermitteln, sowie die fünfte Testprobe zu analysieren und eine fünfte Gerinnungszeit zu ermitteln.

10. Probenanalysevorrichtung nach Anspruch 8, wobei der Prozessor (300) ferner wie folgt konfiguriert ist:

nachdem das inkubierte Patientenplasma aus dem vierten Puffergefäß aspiriert wurde, die Komponente zur Vorbereitung von Testproben (100) so zu steuern, dass das Patientenplasma im vierten Puffergefäß weiterhin bei einer Temperatur von 30 bis 45 °C inkubiert wird, um das Patientenplasma im vierten Puffergefäß für einen erneuten Test verwendbar zu machen; und/oder

nachdem das inkubierte normale Plasma aus dem fünften Puffergefäß aspiriert wurde, die Komponente zur Vorbereitung von Testproben (100) so zu steuern, dass das normale Plasma im fünften Puffergefäß weiterhin bei einer Temperatur von 30 bis 45 °C inkubiert wird, um das normale Plasma im fünften Puffergefäß für einen erneuten Test verwendbar zu machen; und/oder

nachdem das inkubierte Mischplasma aus dem sechsten Puffergefäß aspiriert wurde, die Komponente zur Vorbereitung von Testproben (100) so zu steuern, dass das Mischplasma im sechsten Puffergefäß weiterhin bei einer Temperatur von 30 bis 45 °C inkubiert wird, um das Mischplasma im sechsten Puffergefäß für einen erneuten Test verwendbar zu machen.

11. Probenanalysevorrichtung nach Anspruch 8, wobei eine in das dritte Reaktionsgefäß durch die Komponente zur Vorbereitung von Testproben (100) dosierte Menge des Mischplasmas einer dritten Menge entspricht, eine in das sechste Puffergefäß durch die Komponente zur Vorbereitung von Testproben (100) dosierte Menge des Mischplasmas einer sechsten Menge entspricht, und eine Gesamtdosis des Patientenplasmas in das vierte Puffergefäß sowie des Normalplasmas in das fünfte Puffergefäß durch die Komponente zur Vorbereitung von Testproben (100) einer siebten Menge entspricht; und wobei die siebte Menge größer ist als die dritte Menge und die sechste Menge größer ist als die dritte Menge, vorzugsweise die siebte Menge fünf- bis sechsmal so groß ist wie die dritte Menge, und die sechste Menge fünf- bis sechsmal so groß ist wie die dritte Menge.

12. Die Probenanalysevorrichtung nach Anspruch 8 umfasst ferner eine Wasserzufuhrvorrichtung (500), wobei der Prozessor (300) weiterhin konfiguriert ist, die Wasserzufuhrvorrichtung (500) zu steuern, um während der Inkubation gemäß einem voreingestellten Zeitintervall und einer voreingestellten Wassermenge Wasser in mindestens eines der folgenden Gefäße zu verabreichen: viertes Puffergefäß, fünftes Puffergefäß und sechstes Puffergefäß

13. Probenanalysevorrichtung nach Anspruch 8, wobei ein Tiefen-Durchmesser-Verhältnis des vierten Puffergefäßes größer ist als ein Schwellenwert für das Tiefen-Durchmesser-Verhältnis, das Tiefen-Durchmesser-Verhältnis des fünften Puffergefäßes größer ist als der Schwellenwert für das Tiefen-Durchmesser-Verhältnis, und das Tiefen-Durchmesser-Verhältnis des sechsten Puffergefäßes größer ist als der Schwellenwert für das Tiefen-Durchmesser-Verhältnis; und wobei der Schwellenwert für das Tiefen-Durchmesser-Verhältnis größer als 5 ist.

14. Probenanalysevorrichtung nach Anspruch 8, wobei alle folgenden Gefäße dieselbe Struktur aufweisen: viertes Puffergefäß, fünftes Puffergefäß, sechstes Puffergefäß, drittes Reaktionsgefäß, sechstes Reaktionsgefäß und siebtes Reaktionsgefäß, sodass das vierte, fünfte und sechste Puffergefäß jeweils für eine Inkubation durch die Komponente zur Vorbereitung von Testproben eingeplant und übertragen werden können.

15. Ein Probenanalyseverfahren, das Foglendes umfasst:
Durchführung (S100) eines Sofort-Korrekturtests, der Folgendes umfasst:

die Vorbereitung einer Testprobe für den Sofort-Korrekturtest, wobei die Testprobe durch Mischen einer Probe für den Sofort-Korrekturtest mit einem Reagenz zur Bestimmung der Gerinnungszeit gebildet wird; und
die Erfassung der Testprobe für den Sofort-Korrekturtest zur Ermittlung einer Gerinnungszeit;
Durchführung (S200) eines Inkubations-Korrekturtests, der Folgendes umfasst:

die Vorbereitung einer Testprobe für den Inkubations-Korrekturtest, wobei die Testprobe durch Mischen einer Probe für den Inkubations-Korrekturtest mit dem Reagenz zur Bestimmung der Gerinnungszeit gebildet wird; und
die Erfassung der Testprobe für den Inkubations-Korrekturtest zur Ermittlung einer Gerinnungszeit; wobei
der Sofort-Korrekturtest mindestens einen dritten Gerinnungsfaktor umfasst; wobei bei dem dritten Gerinnungsfaktor die Probe für den Sofort-Korrekturtest ein Mischplasma ist, das durch Mischen von Patientenplasma und Normalplasma gebildet wird, und der dritte Gerinnungsfaktor Folgendes umfasst:

Erfassen von Patientenplasma und Normalplasma, Mischen des Patientenplasmas mit dem Normalplasma zur Bildung des Mischplasmas sowie Mischen des Mischplasmas mit dem Reagenz zur Bestimmung der Gerinnungszeit zur Herstellung einer dritten Testprobe;
Erfassen der dritten Testprobe zur Ermittlung einer dritten Gerinnungszeit;

Wobei der Inkubations-Korrekturtest mindestens einen sechsten und einen siebten Gerinnungsfaktor umfasst;

bei dem sechsten Gerinnungsfaktor die Probe für den Inkubations-Korrekturtest ein Mischplasma ist, das durch Mischen von Patientenplasma und Normalplasma gebildet wird, und der sechste Gerinnungsfaktor Folgendes umfasst:

Erfassen von Patientenplasma und Normalplasma, Mischen des Patientenplasmas mit dem Normalplasma zur Bildung des Mischplasmas, Inkubation des Mischplasmas über eine voreingestellte Dauer unter voreingestellten Bedingungen, und Mischen des inkubierten Mischplasmas mit dem Reagenz zur Bestimmung der Gerinnungszeit zur Herstellung einer sechsten Testprobe; Erfassen der sechsten Testprobe zur Ermittlung einer sechsten Gerinnungszeit; **dadurch gekennzeichnet, dass** bei dem siebten Gerinnungsfaktor die Probe für den Inkubations-Korrekturtest ein Mischplasma ist, das durch Mischen von Patientenplasma und Normalplasma gebildet wird, und der siebte Gerinnungsfaktor Folgendes umfasst:

Erfassen von Patientenplasma und Normalplasma, Inkubation jedes der Patientenplasma und des Normalplasmas über eine voreingestellte Dauer unter voreingestellten Bedingungen, Mischen des inkubierten Patientenplasmas mit dem inkubierten Normalplasma zur Bildung des Mischplasmas sowie Mischen des so gebildeten Mischplasmas mit dem Reagenz zur Bestimmung der Gerinnungszeit zur Herstellung einer siebten Testprobe; und die siebte Testprobe erfassen, um eine siebte Gerinnungszeit zu ermitteln.

## Revendications

1. Appareil d'analyse d'échantillon, comprenant un composant de préparation d'échantillon de test (100), un composant de détection (200) et un processeur (300) relié au composant de préparation d'échantillon de test (100) et au composant de détection (200), où le composant de préparation d'échantillon de test (100) comprend un composant de chargement de récipient de réaction (10), un composant d'alimentation en échantillon (20), un composant de distribution d'échantillon (30), un composant de contenance de réactif (40), un composant de distribution de réactif (60), un composant de réaction (50) et un mécanisme de programmation (70), où :
le composant de contenance de réactif (40) est configuré pour contenir un récipient de réactif, où le récipient de réactif contient un réactif, et le réactif comprend un réactif destiné à déterminer un temps de coagulation ; et le processeur (300) est configuré pour :

commander le composant de chargement de récipient de réaction (10) pour fournir un récipient de réaction et/ou un récipient de tampon ;
commander le composant d'alimentation en échantillon (20) pour fournir un récipient d'échantillon, où le récipient d'échantillon contient un échantillon, et l'échantillon est du plasma de patient ou du plasma normal ;
commander le composant de distribution d'échantillon (30) pour aspirer l'échantillon dans le récipient d'échantillon et distribuer l'échantillon dans le récipient de réaction ou le récipient de tampon ;
commander le composant de distribution de réactif (60) pour aspirer le réactif à partir du récipient de réactif et distribuer le réactif dans le récipient de réaction ;
commander le composant de réaction (50) pour incuber le récipient de réaction et/ou le récipient de tampon contenant l'échantillon, et pour incuber le récipient de réaction contenant l'échantillon et le réactif ;
commander le mécanisme de programmation (70) pour programmer le récipient de réaction et le récipient de tampon, pour programmer le récipient de réaction à transférer vers le composant de réaction (50) et programmer le récipient de tampon à transférer vers le composant de réaction (50) ; et
commander le composant de détection (200), de préférence un composant de détection optique, un composant de détection selon une méthode à doubles billes magnétiques, ou un composant de détection optique et magnétique, pour détecter un échantillon de test formé en mélangeant l'échantillon et le réactif, où le processeur (300) est en outre configuré pour :

commander le composant de distribution d'échantillon (30) pour aspirer le plasma de patient à partir du récipient d'échantillon contenant le plasma de patient et le plasma normal à partir du récipient d'échantillon contenant le plasma normal, et pour distribuer le plasma de patient et le plasma normal dans un troisième récipient de réaction pour former du plasma mixte ; commander le composant de distribution de réactif (60) pour aspirer le réactif destiné à déterminer le temps de coagulation à partir du récipient de réactif, et pour ajouter le réactif destiné à déterminer le temps de coagulation dans le troisième récipient de réaction pour préparer un troisième échantillon de test ;

commander le composant de distribution d'échantillon (30) pour aspirer le plasma de patient à partir du récipient d'échantillon contenant le plasma de patient et le plasma normal à partir du récipient d'échantillon contenant le plasma normal, et pour distribuer le plasma de patient et le plasma normal dans un sixième récipient de tampon pour former du plasma mixte ; commander le composant de réaction (50) pour incuber le plasma mixte dans le sixième récipient de tampon pendant 0,5 à 4 heures à une température de 30 à 45 °C ; commander le mécanisme de programmation (70) pour programmer le sixième récipient de tampon à transférer vers le composant de réaction (50) ; commander le composant de distribution d'échantillon (30) pour aspirer le plasma mixte incubé à partir du sixième récipient de tampon et distribuer le plasma mixte incubé dans un sixième récipient de réaction ; et commander le composant de distribution de réactif (60) pour aspirer le réactif destiné à déterminer le temps de coagulation à partir du récipient de réactif, et pour ajouter le réactif destiné à déterminer le temps de coagulation dans le sixième récipient de réaction pour préparer un sixième échantillon de test ;

**caractérisé en ce que** le processeur (300) est en outre configuré pour :

commander le composant de distribution d'échantillon (30) pour aspirer le plasma de patient à partir du récipient d'échantillon contenant le plasma de patient et distribuer le plasma de patient dans un quatrième récipient de tampon ; commander le composant de réaction (50) pour incuber le plasma de patient dans le quatrième récipient de tampon pendant 0,5 à 4 heures à une température de 30 à 45 °C ; commander le mécanisme de programmation (70) pour programmer le quatrième récipient de tampon à transférer vers le composant de réaction (50) ; commander le composant de distribution d'échantillon (30) pour aspirer le plasma normal à partir du récipient d'échantillon contenant le plasma normal et distribuer le plasma normal dans un cinquième récipient de tampon ; commander le composant de réaction (50) pour incuber le plasma normal dans le cinquième récipient de tampon pendant 0,5 à 4 heures à une température de 30 à 45 °C ; commander le mécanisme de programmation (70) pour programmer le cinquième récipient de tampon à transférer vers le composant de réaction (50) ; commander le composant de distribution d'échantillon (30) pour aspirer le plasma de patient incubé à partir du quatrième récipient de tampon et le plasma normal incubé à partir du cinquième récipient de tampon et distribuer le plasma de patient incubé et le plasma normal incubé dans un septième récipient de réaction ; et commander le composant de distribution de réactif (60) pour aspirer le réactif destiné à déterminer le temps de coagulation à partir du récipient de réactif, et pour ajouter le réactif destiné à déterminer le temps de coagulation dans le septième récipient de réaction pour préparer un septième échantillon de test ; et

commander le composant de détection (200) pour détecter le troisième échantillon de test pour obtenir un troisième temps de coagulation, détecter le sixième échantillon de test pour obtenir un sixième temps de coagulation, et détecter le septième échantillon de test pour obtenir un septième temps de coagulation.

2. Appareil d'analyse d'échantillon selon la revendication 1, dans lequel le processeur (300) est en outre configuré pour :

commander le composant de distribution d'échantillon (30) pour aspirer le plasma de patient incubé à partir du quatrième récipient de tampon et distribuer le plasma de patient incubé dans un quatrième récipient de réaction ; commander le composant de distribution de réactif (60) pour aspirer le réactif destiné à déterminer le temps de coagulation à partir du récipient de réactif et ajouter le réactif destiné à déterminer le temps de coagulation dans le quatrième récipient de réaction pour préparer un quatrième échantillon de test ;

commander le composant de distribution d'échantillon (30) pour aspirer le plasma normal incubé à partir du cinquième récipient de tampon et distribuer le plasma normal incubé dans un cinquième récipient de réaction ; commander le composant de distribution de réactif (60) pour aspirer le réactif destiné à déterminer le temps de coagulation à partir du récipient de réactif et ajouter le réactif destiné à déterminer le temps de coagulation dans le cinquième récipient de réaction pour préparer un cinquième échantillon de test ; et

commander le composant de détection (200) pour détecter le quatrième échantillon de test pour obtenir un quatrième temps de coagulation, et détecter le cinquième échantillon de test pour obtenir un cinquième temps de coagulation.

3. Appareil d'analyse d'échantillon selon la revendication 1 ou 2, dans lequel le processeur (300) est en outre configuré pour :

après que le composant de distribution d'échantillon (30) a aspiré le plasma de patient incubé à partir du quatrième récipient de tampon, commander le composant de réaction (50) pour continuer d'incuber le plasma de patient dans le quatrième récipient de tampon à une température de 30 à 45 °C, pour permettre au plasma de patient dans le quatrième récipient de tampon d'être utilisé pour un nouveau test ; et/ou

après que le composant de distribution d'échantillon (30) a aspiré le plasma normal incubé à partir du cinquième récipient de tampon, commander le composant de réaction (50) pour continuer d'incuber le plasma normal dans le cinquième récipient de tampon à une température de 30 à 45 °C, pour permettre au plasma normal dans le cinquième récipient de tampon d'être utilisé pour un nouveau test ; et/ou

après que le composant de distribution d'échantillon (30) a aspiré le plasma mixte incubé à partir du sixième récipient de tampon, commander le composant de réaction (50) pour continuer d'incuber le plasma mixte dans le sixième récipient de tampon à une température de 30 à 45 °C, pour permettre au plasma mixte dans le sixième récipient de tampon d'être utilisé pour un nouveau test.

4. Appareil d'analyse d'échantillon selon la revendication 1, dans lequel une quantité du plasma mixte distribué dans le troisième récipient de réaction par le composant de distribution d'échantillon (30) est une troisième quantité, une quantité du plasma mixte distribué dans le sixième récipient de tampon par le composant de distribution d'échantillon (30) est une sixième quantité, et une quantité totale du plasma de patient distribué dans le quatrième récipient de tampon et du plasma normal distribué dans le cinquième récipient de tampon par le composant de distribution d'échantillon (30) est une septième quantité ; et où la septième quantité est supérieure à la troisième quantité, et la sixième quantité est supérieure à la troisième quantité, de préférence, la septième quantité est de cinq à six fois la troisième quantité, et la sixième quantité est de cinq à six fois la troisième quantité.

5. Appareil d'analyse d'échantillon selon la revendication 1, comprenant en outre un appareil de rajout d'eau (500), et le processeur (300) est en outre configuré pour commander l'appareil de rajout d'eau (500) pour distribuer de l'eau dans au moins l'un du quatrième récipient de tampon, du cinquième récipient de tampon et du sixième récipient de tampon durant l'incubation selon un intervalle de temps prédéfini et une quantité prédéfinie de rajout d'eau.

6. Appareil d'analyse d'échantillon selon la revendication 1, dans lequel un rapport profondeur-diamètre du quatrième récipient de tampon est supérieur à un seuil de rapport profondeur-diamètre, un rapport profondeur-diamètre du cinquième récipient de tampon est supérieur au seuil de rapport profondeur-diamètre, et un rapport profondeur-diamètre du sixième récipient de tampon est supérieur au seuil de rapport profondeur-diamètre ; et où le seuil de rapport profondeur-diamètre est supérieur à 5.

7. Appareil d'analyse d'échantillon selon la revendication 1, dans lequel le quatrième récipient de tampon, le cinquième récipient de tampon, le sixième récipient de tampon et le septième récipient de tampon ont tous la même structure, de sorte que le quatrième récipient de tampon, le cinquième récipient de tampon, et le sixième récipient de tampon sont en mesure d'être programmés pour être transférés dans le composant de réaction (50) pour incubation.

8. Appareil d'analyse d'échantillon, comprenant :
un composant de préparation d'échantillon de test (100), un composant de détection (200) et un processeur (300), où le processeur (300) est configuré pour commander le composant de préparation d'échantillon de test (100) pour :

aspirer du plasma de patient à partir d'un récipient d'échantillon contenant le plasma de patient et du plasma normal à partir d'un récipient d'échantillon contenant le plasma normal respectivement, distribuer le plasma de patient et le plasma normal dans un troisième récipient de réaction pour former du plasma mixte, et ajouter un réactif destiné à déterminer un temps de coagulation dans le troisième récipient de réaction pour préparer un troisième échantillon de test ;

aspirer le plasma de patient à partir du récipient d'échantillon contenant le plasma de patient et le plasma normal à partir du récipient d'échantillon contenant le plasma normal respectivement, distribuer le plasma de patient et le plasma normal dans un sixième récipient de tampon pour former du plasma mixte, incuber le plasma mixte dans le sixième récipient de tampon pendant 0,5 à 4 heures à une température de 30 à 45 °C, aspirer le plasma mixte incubé à partir du sixième récipient de tampon et distribuer le plasma mixte incubé dans un sixième récipient de réaction, et ajouter le réactif destiné à déterminer le temps de coagulation dans le sixième récipient de réaction pour préparer un sixième échantillon de test ;

**caractérisé en ce que** le processeur (300) est en outre configuré pour :

commander le composant de préparation d'échantillon de test (100) pour aspirer le plasma de patient à partir du récipient d'échantillon contenant le plasma de patient et distribuer le plasma de patient dans un quatrième récipient de tampon, incuber le plasma de patient dans le quatrième récipient de tampon pendant 0,5 à 4 heures à une température de 30 à 45 °C, aspirer le plasma normal à partir du récipient d'échantillon contenant le plasma normal et distribuer le plasma normal dans un cinquième récipient de tampon, incuber le plasma normal dans le cinquième récipient de tampon pendant 0,5 à 4 heures à une température de 30 à 45 °C, aspirer le plasma de patient incubé à partir du quatrième récipient de tampon et le plasma normal incubé à partir du cinquième récipient de tampon respectivement et distribuer le plasma de patient incubé et le plasma normal incubé dans un septième récipient de réaction, et ajouter le réactif destiné à déterminer le temps de coagulation dans le septième récipient de réaction pour préparer un septième échantillon de test ; et commander le composant de détection (200), de préférence un composant de détection optique, un composant de détection selon une méthode à doubles billes magnétiques, ou un composant de détection optique et magnétique, pour détecter le troisième échantillon de test pour obtenir un troisième temps de coagulation, détecter le sixième échantillon de test pour obtenir un sixième temps de coagulation, et détecter le septième échantillon de test pour obtenir un septième temps de coagulation.

9. Appareil d'analyse d'échantillon selon la revendication 8, dans lequel le processeur (300) est en outre configuré pour commander le composant de préparation d'échantillon de test (100) pour : aspirer le plasma de patient incubé à partir du quatrième récipient de tampon et distribuer le plasma de patient incubé dans un quatrième récipient de réaction, et ajouter le réactif destiné à déterminer le temps de coagulation dans le quatrième récipient de réaction pour préparer un quatrième échantillon de test ; et le processeur (300) est en outre configuré pour commander le composant de préparation d'échantillon de test (100) pour : aspirer le plasma normal incubé à partir du cinquième récipient de tampon et distribuer le plasma normal incubé dans un cinquième récipient de réaction, et ajouter le réactif destiné à déterminer le temps de coagulation dans le cinquième récipient de réaction pour préparer un cinquième échantillon de test ; et
le processeur (300) est en outre configuré pour commander le composant de détection (200) pour détecter le quatrième échantillon de test pour obtenir un quatrième temps de coagulation, et détecter le cinquième échantillon de test pour obtenir un cinquième temps de coagulation.

10. Appareil d'analyse d'échantillon selon la revendication 8, dans lequel le processeur (300) est en outre configuré pour :

après avoir aspiré le plasma de patient incubé à partir du quatrième récipient de tampon, commander le composant de préparation d'échantillon de test (100) pour continuer d'incuber le plasma de patient dans le quatrième récipient de tampon à une température de 30 à 45 °C, pour permettre au plasma de patient dans le quatrième récipient de tampon d'être utilisé pour un nouveau test ; et/ou
après avoir aspiré le plasma normal incubé à partir du cinquième récipient de tampon, commander le composant de préparation d'échantillon de test (100) pour continuer d'incuber le plasma normal dans le cinquième récipient de tampon à une température de 30 à 45 °C, pour permettre au plasma normal dans le cinquième récipient de tampon d'être utilisé pour un nouveau test ; et/ou
après avoir aspiré le plasma mixte incubé à partir du sixième récipient de tampon, commander le composant de préparation d'échantillon de test (100) pour continuer d'incuber le plasma mixte dans le sixième récipient de tampon à une température de 30 à 45 °C, pour permettre au plasma mixte dans le sixième récipient de tampon d'être utilisé pour un nouveau test.

11. Appareil d'analyse d'échantillon selon la revendication 8, dans lequel une quantité du plasma mixte distribué dans le troisième récipient de réaction par le composant de préparation d'échantillon de test (100) est une troisième quantité, une quantité du plasma mixte distribué dans le sixième récipient de tampon par le composant de préparation d'échantillon de test (100) est une sixième quantité, et une quantité totale du plasma de patient distribué dans le quatrième récipient de tampon et du plasma normal distribué dans le cinquième récipient de tampon par le composant de préparation d'échantillon de test (100) est une septième quantité ; et où la septième quantité est supérieure à la troisième quantité, et la sixième quantité est supérieure à la troisième quantité, de préférence, la septième quantité est de cinq à six fois la troisième quantité, et la sixième quantité est de cinq à six fois la troisième quantité.

12. Appareil d'analyse d'échantillon selon la revendication 8, comprenant en outre un appareil de rajout d'eau (500), où le processeur (300) est en outre configuré pour commander l'appareil de rajout d'eau (500) pour distribuer de l'eau dans au moins l'un du quatrième récipient de tampon, du cinquième récipient de tampon et du sixième récipient de tampon durant l'incubation selon un intervalle de temps prédéfini et une quantité prédéfinie de rajout d'eau.

13. Appareil d'analyse d'échantillon selon la revendication 8, dans lequel un rapport profondeur-diamètre du quatrième récipient de tampon est supérieur à un seuil de rapport profondeur-diamètre, un rapport profondeur-diamètre du cinquième récipient de tampon est supérieur au seuil de rapport profondeur-diamètre, et un rapport profondeur-diamètre du sixième récipient de tampon est supérieur au seuil de rapport profondeur-diamètre ; et où le seuil de rapport profondeur-diamètre est supérieur à 5.

14. Appareil d'analyse d'échantillon selon la revendication 8, dans lequel le quatrième récipient de tampon, le cinquième récipient de tampon, le sixième récipient de tampon et le septième récipient de tampon ont tous la même structure, de sorte que le quatrième récipient de tampon, le cinquième récipient de tampon, et le sixième récipient de tampon sont en mesure d'être programmés pour être transférés dans le composant de préparation d'échantillon de test pour incubation.

15. Procédé d'analyse d'échantillon, comprenant les étapes consistant à :

réaliser (S100) un test de correction immédiat, où le test de correction immédiat comprend les étapes consistant à :

préparer un échantillon de test destiné au test de correction immédiat, où l'échantillon de test destiné au test de correction immédiat est formé en mélangeant un échantillon destiné au test de correction immédiat et un réactif destiné à déterminer un temps de coagulation ; et
détecter l'échantillon de test destiné au test de correction immédiat pour obtenir un temps de coagulation ;

réaliser (S200) un test de correction d'incubation, où le test de correction d'incubation comprend les étapes consistant à :

préparer un échantillon de test destiné au test de correction d'incubation, où l'échantillon de test destiné au test de correction d'incubation est formé en mélangeant un échantillon destiné au test de correction d'incubation et un réactif destiné à déterminer le temps de coagulation ; et
détecter l'échantillon de test destiné au test de correction d'incubation pour obtenir un temps de coagulation ;

où
le test de correction immédiat comprend au moins un troisième élément de coagulation ; dans le troisième élément de coagulation, l'échantillon destiné au test de correction immédiat est du plasma mixte formé en mélangeant du plasma de patient et du plasma normal, et le troisième élément de coagulation comprend :
le fait d'acquérir le plasma de patient et le plasma normal, de mélanger le plasma de patient et le plasma normal pour former le plasma mixte, et de mélanger le plasma mixte et le réactif destiné à déterminer le temps de coagulation pour préparer un troisième échantillon de test ;
détecter le troisième échantillon de test pour obtenir un troisième temps de coagulation ; où
le test de correction d'incubation comprend au moins un sixième élément de coagulation et un septième élément de coagulation ;
dans le sixième élément de coagulation, l'échantillon destiné au test de correction d'incubation est du plasma mixte formé en mélangeant le plasma de patient et le plasma normal, et le sixième élément de coagulation comprend :

le fait d'acquérir le plasma de patient et le plasma normal, de mélanger le plasma de patient et le plasma normal pour former le plasma mixte, d'incuber le plasma mixte pendant une durée prédéfinie sous une condition prédéfinie, et de mélanger le plasma mixte incubé et le réactif destiné à déterminer le temps de coagulation pour préparer un sixième échantillon de test ;
détecter le sixième échantillon de test pour obtenir un sixième temps de coagulation ;
**caractérisé en ce que**
dans le septième élément de coagulation, l'échantillon destiné au test de correction d'incubation est du plasma mixte formé en mélangeant le plasma de patient et le plasma normal, et le septième élément de coagulation comprend :

le fait d'acquérir le plasma de patient et le plasma normal, d'incuber chacun du plasma de patient et du plasma normal pendant une durée prédéfinie sous une condition prédéfinie, de mélanger le plasma de patient incubé et le plasma normal incubé pour former le plasma mixte, et de mélanger le plasma mixte formé en mélangeant le plasma de patient incubé et le plasma normal incubé et le réactif destiné à déterminer le temps de coagulation pour préparer un septième échantillon de test ; et
détecter le septième échantillon de test pour obtenir un septième temps de coagulation.

Testing sample preparation
component 100

Detection component
200

**FIG. 1**

Testing sample
preparation component
100

Detection
component 200

Processor 300

Display 400

**FIG. 2**

**FIG. 3**

**FIG. 4A**

**FIG. 4B**

**FIG. 5**

| Type | Test | Brief introduction of test process |
|---|---|---|
| Immediate correction | APTT1 | An APTT result is obtained by directly testing patient plasma |
| | APTT2 | An APTT result is obtained by directly testing normal plasma |
| | APTT3 | Patient plasma and normal plasma are mixed in a ratio of 1:1 (or 1:4, a plurality of ratios may be adopted, but the ratio adopted by APTT6 must be included), and a test is performed to obtain an APTT result |
| Incubation correction | APTT4 | The patient plasma is independently incubated for 2 hours at 37℃, and then a test is performed to obtain an APTT result |
| | APTT5 | The normal plasma is independently incubated for 2 hours at 37℃, and then a test is performed to obtain an APTT result |
| | APTT6 | The patient plasma and the normal plasma are mixed in the ratio of 1:1 (or 1:4, one ratio is adopted generally), the mixed plasma is incubated for 2 hours at 37℃, and then a test is performed to obtain an APTT result |
| | APTT7 | The patient plasma is incubated for 2 hours at 37℃ and the normal plasma is incubated for 2 hours at 37℃, then the incubated patient plasma and the normal plasma are mixed (the ratio needs to be consistent with APTT6), and then a test is performed to obtain an APTT result |

**FIG. 6**

**FIG. 7**

**FIG. 8**

Remaining liquid volume of the incubation sample

**FIG. 9**

EP 4 227 687 B1

**FIG. 10**

| | Incubation liquid volume/uL | Water replenishing medium | Water replenishing amount /uL | Water replenishing interval /min |
|---|---|---|---|---|
| First condition | 300 | Purified water | 9 | 12 |
| Second condition | 300 | Purified water | 15 | 30 |
| Third condition | 50 | Purified water | 15 | 12 |

**FIG. 11**

**FIG. 12**

|  | Test method | APTT1 | APTT2 | APTT3 | APTT4 | APTT5 | APTT6 | APTT7 |
|---|---|---|---|---|---|---|---|---|
| First sample Hereditary hemophilia A | Instrument | 76.18 | 33.44 | 28.32 | 81.00 | 31.34 | 29.19 | 31.94 |
|  | Manual | 78.18 | 33.84 | 29.15 | 87.82 | 32.76 | 30.09 | 61.65 |
| Second sample LA positive | Instrument | 111.26 | 64.57 | 28.70 | 121.94 | 70.21 | 28.70 | 71.08 |
|  | Manual | 111.45 | 65.86 | 29.01 | 126.68 | 69.24 | 28.62 | 69.11 |
| Third sample Acquired hemophilia A | Instrument | 77.25 | 32.09 | 28.33 | 77.25 | 46.6 | 29.40 | 32.40 |
|  | Manual | 76.11 | 31.79 | 29.20 | 78.93 | 48.54 | 28.45 | 32.37 |

**FIG. 13**

FIG. 14

FIG. 15

Test report for APTT mixed plasma correction test (template)

| Test item | Test result | Reference range |
|---|---|---|
| APTT1 (Patient) | | Reference range |
| APTT2 (Normal pooled plasma) | | Reference range |
| APTT3 (Immediate test for mixed plasma) | | – |
| APTT4 (Patient plasma is incubated for 2 hours) | | – |
| APTT5 (Normal pooled plasma is incubated for 2 hours) | | – |
| APTT6 (1:1 mixed plasma is incubated for 2 hours) | | – |
| APTT7 (1:1 mixed plasma after being incubated for 2 hours respectively) | | – |
| Rosner Index(RI) | | <10: Coagulation factor deficiency; >15: Inhibitor 10-15: Gray area |
| Prolonged time after incubation (APTT 6-APTT 7) | | ≤3s,Time-independent inhibitor >3s,Time-dependent inhibitor |

Result determination

| | Immediate test | Test after 2-hour incubation at 37℃ |
|---|---|---|
| Coagulation factor deficiency | APTT3 corrected | APTT6 and APTT7 corrected |
| Time and temperature-dependent inhibitor | APTT3 corrected | APTT7 corrected, and the APTT6 is prolonged by a cutoff value (3s) than APTT7 |
| | APTT3 uncorrected | APTT6 and APTT7 uncorrected, APTT7 corrected, and the APTT6 is prolonged by a cutoff value (3s) than APTT7 |
| Time and temperature-independent inhibitor | APTT3 uncorrected | APTT6 and APTT7 uncorrected, APTT7 corrected, and the APTT6 is prolonged by no more than the cutoff value (3s) than APTT7 |

Remark:

1.A mixed plasma correction test may be considered to be started for unexplained APTT prolonging;

2.Pre-analysis errors, such as insufficient or excessive collection, clots, contamination by other anticoagulants or coagulants, may affect APTT results, and the mixed plasma correction test should be performed after excluding these influencing factors;

3.The mixed plasma correction test is used for preliminary screening of the reason for APTT rise, which cannot ensure the accuracy of 100%, and actual clinical situation and relevant confirmation tests need to be combined to analyze comprehensively.

# FIG. 16

Test report for APTT mixed plasma correction test (example)

| Test item | Test result | Reference range |
|---|---|---|
| APTT1 (Patient) | | Reference range |
| APTT2 (Normal pooled plasma) | | Reference range |
| APTT3 (Immediate test for mixed plasma) | | – |
| APTT4 (Patient plasma is incubated for 2 hours) | | – |
| APTT5 (Normal pooled plasma is incubated for 2 hours) | | – |
| APTT6 (1:1 mixed plasma is incubated for 2 hours) | | – |
| APTT7 (1:1 mixed plasma after being incubated for 2 hours respectively) | | – |
| Rosner Index(RI) | | <10: Coagulation factor deficiency; >15: Inhibitor 10-15: Gray area |
| Prolonged time after incubation (APTT 6-APTT 7) | | ≤3s,Time-independent inhibitor >3s,Time-dependent inhibitor |
| APTTD (APTT correction difference) APTT 6-APTT3 | | ≤1.6s,Coagulation factor deficiency >1.6s,Existence of an inhibitor |
| APTTCI (APTT correction index) APTT1×APTT3×APTT6× APTTD^3/10^6 | | ≤0.55,Coagulation factor deficiency >0.55,Existence of an inhibitor |

Result determination

| | Immediate test | Test after 2-hour incubation at 37℃ |
|---|---|---|
| Coagulation factor deficiency | APTT3 corrected | APTT6 and APTT7 corrected |
| Time and temperature-dependent inhibitor | APTT3 corrected | APTT7 corrected, and the APTT6 is prolonged by a cutoff value (3s) than APTT7 |
| | APTT3 uncorrected | APTT6 and APTT7 uncorrected, APTT7 corrected, and the APTT6 is prolonged by a cutoff value (3s) than APTT7 |
| Time and temperature-independent inhibitor | APTT3 uncorrected | APTT6 and APTT7 uncorrected, APTT7 corrected, and the APTT6 is prolonged by no more than the cutoff value (3s) than APTT7 |

Remark:

1.A mixed plasma correction test may be considered to be started for unexplained APTT prolonging;

2.Pre-analysis errors, such as insufficient or excessive collection, clots, contamination by other anticoagulants or coagulants, may affect APTT results, and the mixed plasma correction test should be performed after excluding these influencing factors;

3.The mixed plasma correction test is used for preliminary screening of the reason for APTT rise, which cannot ensure the accuracy of 100%, and actual clinical situation and relevant confirmation tests need to be combined to analyze comprehensively.

# FIG. 17

**FIG. 18**

**FIG. 19**

**FIG. 20**

**FIG. 21**

An immediate correction test is performed, and the immediate correction test includes a third coagulation item

S100

An incubation correction test is performed, and the incubation correction test includes a sixth coagulation item and a seventh coagulation item

S200

**FIG. 22**

**REFERENCES CITED IN THE DESCRIPTION**

**Patent documents cited in the description**

- US 10578628 B2

- US 11155542 B2